# EUROPEAN PATENT APPLICATION

(11) **EP 4 643 886 A1**
(43) Date of publication of application: **05.11.2025**
(21) Application number: 23910734.5
(22) Date of filing: 27.12.2023
(51) Int. Cl.: A61K 47/68, A61K 35/17, A61P 35/00, C12N 5/0783

(54) **ANTI-CD33/CLL1 BISPECIFIC ANTIBODY-NATURAL KILLER CELL CONJUGATE AND USE THEREOF**

(30) Priority: 30.12.2022 CN 202211728567
(71) Applicant: IMBIORAY (HANGZHOU) BIOMEDICINE CO., LTD., Hangzhou, Zhejiang 310052 (CN)
(72) Inventor: MIAO, Zhenwei, Hangzhou, Zhejiang 310052 (CN); ZHENG, Chen, Hangzhou, Zhejiang 310052 (CN); BIAN, Jinduo, Hangzhou, Zhejiang 310052 (CN); WU, Liqin, Hangzhou, Zhejiang 310052 (CN); SANG, Aowen, Hangzhou, Zhejiang 310052 (CN); SONG, Yan, Hangzhou, Zhejiang 310052 (CN); WANG, Guojun, Hangzhou, Zhejiang 310052 (CN); LU, Huan, Hangzhou, Zhejiang 310052 (CN); WANG, Cong, Hangzhou, Zhejiang 310052 (CN); LIU, Shizhou, Hangzhou, Zhejiang 310052 (CN); CHEN, Mingyue, Hangzhou, Zhejiang 310052 (CN); MEI, Shuang, Hangzhou, Zhejiang 310052 (CN)
(74) Representative: Nederlandsch Octrooibureau
(86) International application number: PCT/CN2023/142402
(87) International publication number: WO 2024/140816

(57) **Abstract**

The present application provides an anti-CD33/CLL1 bispecific antibody-natural killer cell conjugate, a pharmaceutical composition comprising the conjugate, medical and pharmaceutical use of the conjugate, and a preparation method therefor.

## Description

### CROSS-REFERENCE TO RELATED APPLICATION

This application claims priority to Chinese Patent Application No. 202211728567.1, filed on December 30, 2022, the entire contents of which are incorporated herein by reference in its entirety.

### TECHNICAL FIELD

The present application relates generally to the field of biopharmaceuticals, and more particularly, to an anti-CD33/CLL1 bispecific antibody-natural killer cell conjugate, a pharmaceutical composition comprising the conjugate, medical and pharmaceutical use of the conjugate, and a preparation method therefor, and the like.

### BACKGROUND

Acute myeloid leukemia (AML) is a highly heterogeneous group of malignant clonal proliferative diseases of myeloid primitive cells in the hematopoietic system, which are transformed from the malignant transformation of myeloid hematopoietic progenitor cells at different stages of differentiation and development.

In recent years, with the increase in the proportion of aged population, the incidence of AML has been rising, and the proportion of relapsed and refractory AML has also been gradually increasing. Although 60%~80% of adult AML patients can receive complete remission (CR) through a first induction chemotherapy, and about 30% of patients then undergo a consolidation therapy and allogeneic bone marrow transplantation to achieve the goal of complete cure, there are still 10%~30% of patients who fail to achieve CR after the induction therapy and suffer from primary refractory AML. Relapses eventually occur in about half of young patients and up to 80%~90% of elderly patients.

Primary principles for the treatment of relapsed/refractory acute myeloid leukemia include the following. 1) For patients who can tolerate intensive chemotherapies, it is recommended to administrate salvage treatments with combination chemotherapies including the FLAG regimen (fludarabine, cytarabine, and granulocyte colony-stimulating factor G-CSF), the CLAG regimen (cladribine, cytarabine, and G-CSF), or intermediate (large) dose of cytarabine (IDAC/HiDAC), etc. If the patients achieve complete remission, allogeneic bone marrow transplantation can be performed. 2) For patients who cannot tolerate intensive chemotherapies, Venentoclax (VEN) combined with Azacitidine (AZA), or low-dose cytarabine (LDAC) combination therapy is administrated. Depending on whether the patients have genetic mutations in FMS-like tyrosine kinase 3 (FLT3), isocitrate dehydrogenase 1/2 (IDH1/2) and other gene mutations, corresponding targeted drugs such as gilteritinib, ivosidenib can also be administrated. Usually, the incidence of gene mutations in such as FLT3 and IDH1/2 is low in AML, and is about 5%~15%. The above treatment regimens can also be adjusted accordingly based on the prognosis determined from the patient's karyotype status, for example, it is recommended to apply the allogeneic bone marrow to populations with medium and high risks.

Relapsed/refractory acute myeloid leukemia has a poor prognosis. According to literature, the complete remission rate of patients with relapsed/refractory acute myeloid leukemia (able to tolerate intensive chemotherapies) is 20%~30% after a chemotherapy, with a median overall survival (OS) of 5~8 months. In order to increase the remission rate and prolong the survival of patients, there are many clinical trials ongoing or in progress worldwide. For example, a multicenter clinical study of Uproleselan (GMI-1271, APL-106) combination chemotherapy is being carried out globally, and a bridging study is being conducted in Chinese patients at the same time. According to the published results of the second phase of the clinical trial, the combination therapy achieves a composite complete remission rate (including complete remission or complete remission with incomplete hematological remission, CR/CRi) of 41%, and an OS of 8.8 months.

In addition to traditional chemotherapies and hematopoietic stem cell transplantation, reported cell immunotherapies include: a) autologous tumor-infiltrating lymphocyte (TIL) therapy; b) NK cell immunotherapy; c) dendritic cell and cytokine-induced killer cell (DC-CIK) therapy; d) macrophage activation chemotherapy; and e) chimeric antigen receptor T cell (CAR-T) immunotherapy. So far, multiple clinical studies of CAR-T and NK cell therapies on AML have been carried out , but no products have been approved.

There is an urgent need in the art for more therapies for acute myeloid leukemia (AML).

### SUMMARY

In a first aspect, the present application provides an antibody-natural killer cell (NK cell) conjugate, wherein the antibody is a bispecific antibody comprising a CD33 antigen-binding fragment and a CLL1 antigen-binding fragment, and the bispecific antibody is conjugated to the NK cell via a linker.

In a second aspect, the present application provides a cell population comprising the antibody-natural killer cell (NK cell) conjugate of the first aspect.

In a third aspect, the present application provides a pharmaceutical composition comprising the antibody-natural killer cell (NK cell) conjugate of the first aspect or the cell population of the second aspect, and a pharmaceutically acceptable carrier.

In a fourth aspect, the present application provides use of the antibody-natural killer cell (NK cell) conjugate of the first aspect or the cell population of the second aspect in the manufacture of a medicament for the treatment of acute myeloid leukemia (AML) in an individual.

In a fifth aspect, the present application provides a method of treating acute myeloid leukemia (AML) in an individual, the method comprising administering to the individual an effective amount of the antibody-natural killer cell (NK cell) conjugate of the first aspect or the cell population of the second aspect or the pharmaceutical composition of the third aspect.

As non-limiting examples, the present application provides the following embodiments.
1. An antibody-natural killer cell (NK cell) conjugate, wherein the antibody is a bispecific antibody comprising a CD33 antigen-binding fragment and a CLL1 antigen-binding fragment, and the bispecific antibody is conjugated to the NK cell via a linker.
2. The antibody-natural killer cell (NK cell) conjugate of embodiment 1, wherein the CD33 antigen-binding fragment comprises:
   HCDR1 as set forth in SEQ ID NO:9 or having at least 80%, 85%, 90%, 95% or 99% identity to the sequence set forth in SEQ ID NO:9,
   HCDR2 as set forth in SEQ ID NO:10 or having at least 80%, 85%, 90%, 95% or 99% identity to the sequence set forth in SEQ ID NO:10,
   HCDR3 as set forth in SEQ ID NO:11 or having at least 80%, 85%, 90%, 95% or 99% identity to the sequence set forth in SEQ ID NO:11,
   LCDR1 as set forth in SEQ ID NO:12 or having at least 80%, 85%, 90%, 95% or 99% identity to the sequence set forth in SEQ ID NO:12,
   LCDR2 as set forth in SEQ ID NO: 13 or having at least 80%, 85%, 90%, 95% or 99% identity to the sequence set forth in SEQ ID NO:13, and
   LCDR3 as set forth in SEQ ID NO: 14 or having at least 80%, 85%, 90%, 95% or 99% identity to the sequence set forth in SEQ ID NO:14; and
   wherein the amino acid sequences of the HCDRs and the LCDRs are defined according to Kabat.
3. The antibody-natural killer cell (NK cell) conjugate of embodiment 1 or 2, wherein the amino acid sequence of the heavy chain variable region of the CD33 antigen-binding fragment is set forth in SEQ ID NO:5 or has at least 80%, 85%, 90%, 95% or 99% identity to the sequence set forth in SEQ ID NO:5, and the amino acid sequence of the light chain variable region is set forth in SEQ ID NO:7 or has at least 80%, 85%, 90%, 95% or 99% identity to the sequence set forth in SEQ ID NO:7.
4. The antibody-natural killer cell (NK cell) conjugate of any one of embodiments 1-3, wherein the CLL1 antigen-binding fragment comprises:
   HCDR1 as set forth in SEQ ID NO: 15 or having at least 80%, 85%, 90%, 95% or 99% identity to the sequence set forth in SEQ ID NO:15,
   HCDR2 as set forth in SEQ ID NO: 16 or having at least 80%, 85%, 90%, 95% or 99% identity to the sequence set forth in SEQ ID NO: 16,
   HCDR3 as set forth in SEQ ID NO:17 or having at least 80%, 85%, 90%, 95% or 99% identity to the sequence set forth in SEQ ID NO:17,
   LCDR1 as set forth in SEQ ID NO:18 or having at least 80%, 85%, 90%, 95% or 99% identity to the sequence set forth in SEQ ID NO:18,
   LCDR2 as set forth in SEQ ID NO: 19 or having at least 80%, 85%, 90%, 95% or 99% identity to the sequence set forth in SEQ ID NO:19, and
   LCDR3 as set forth in SEQ ID NO:20 or having at least 80%, 85%, 90%, 95% or 99% identity to the sequence set forth in SEQ ID NO:20; and
   wherein the amino acid sequences of the HCDRs and the LCDRs are defined according to Kabat.
5. The antibody-natural killer cell (NK cell) conjugate of any one of embodiments 1-4, wherein the amino acid sequence of the heavy chain variable region of the CLL1 antigen-binding fragment is set forth in SEQ ID NO:1 or has at least 80%, 85%, 90%, 95% or 99% identity to the sequence set forth in SEQ ID NO:1, and the amino acid sequence of the light chain variable region is set forth in SEQ ID NO:3 or has at least 80%, 85%, 90%, 95% or 99% identity to the sequence set forth in SEQ ID NO:3.
6. The antibody-natural killer cell (NK cell) conjugate of any one of embodiments 1-5, wherein the forms of the CD33 antigen-binding fragment and the CLL1 antigen-binding fragment are independently selected from a single chain antibody (scFv) or a Fab fragment.
7. The antibody-natural killer cell (NK cell) conjugate of any one of embodiments 1-6, wherein the bispecific antibody comprises a CD33 antibody arm comprising the CD33 antigen-binding fragment, and a CLL1 antibody arm comprising the CLL1 antigen-binding fragment, wherein
   the CD33 antibody arm comprises a heavy chain variable region set forth in SEQ ID NO:5, a heavy chain constant region set forth in SEQ ID NO:6, a light chain variable region set forth in SEQ ID NO:7, and a light chain constant region set forth in SEQ ID NO:8; and/or
   the CLL1 antibody arm comprises a heavy chain variable region set forth in SEQ ID NO: 1, a heavy chain constant region set forth in SEQ ID NO:2, a light chain variable region set forth in SEQ ID NO:3, and a light chain constant region set forth in SEQ ID NO:4.
8. The antibody-natural killer cell (NK cell) conjugate of any one of embodiments 1-7, wherein:
   the bispecific antibody is an intact antibody; and/or
   the bispecific antibody is a humanized antibody or a fully human antibody; and/or
   the bispecific antibody is a monoclonal antibody; and/or
   the bispecific antibody is of IgG1, IgG2 or IgG4 isotype; and/or
   the bispecific antibody comprises a light chain constant region of κ subtype.
9. The antibody-natural killer cell (NK cell) conjugate of any one of embodiments 1-8, wherein the NK cell is CD16⁺ and/or NKG2D⁺, preferably CD16⁺NKG2D⁺.
10. The antibody-natural killer cell (NK cell) conjugate of any one of embodiments 1-9, wherein the conjugate comprises at least 90% of CD16⁺NKG2D⁺NK cells, preferably, meanwhile the conjugate comprises at least 90% of CD56⁺NK cells.
11. The antibody-natural killer cell (NK cell) conjugate of any one of embodiments 1-10, wherein
   the NK cell is obtained from *in vitro* culture and expansion of NK cells derived from peripheral blood mononuclear cells (PBMCs); or
   the NK cell is obtained from *in vitro* culture and expansion of NK cells derived from umbilical cord blood; or
   the NK cell is obtained from *in vitro* induction, culture and expansion of inducible pluripotent stem cells (iPSCs) or mesenchymal stem cells (ESCs).
12. The antibody-natural killer cell (NK cell) conjugate of any one of embodiments 1-11, wherein the bispecific antibody is conjugated to the NK cell via a click chemical reaction of linkers.
13. The antibody-natural killer cell (NK cell) conjugate of embodiment 12, wherein the bispecific antibody is conjugated to the NK cell via a first linker conjugated to the bispecific antibody and a second linker conjugated to the NK cell, with the first linker and the second linker conjugated to each other to form the antibody-NK cell conjugate.
14. The antibody-natural killer cell (NK cell) conjugate of embodiment 13, wherein the first linker is an active ester capable of forming a conjugation to a lysine residue of the antibody by a reaction converting an ester bond into an amide bond, e.g., the active ester is a pentafluorophenyl ester, such as pentafluorophenyl piperidinate.
15. The antibody-natural killer cell (NK cell) conjugate of embodiment 14, wherein the first linker further comprises a carbon-carbon triple bond structure capable of undergoing a cyclization reaction with an azide group to form a five-membered ring of triazole, e.g., the carbon-carbon triple bond structure is an octyne group.
16. The antibody-natural killer cell (NK cell) conjugate of embodiment 15, wherein the first linker is a dibenzoazacyclooctyne-glutaryl-amino polyethylene glycol-pentafluorophenyl acetylpiperidinate having the following structure: 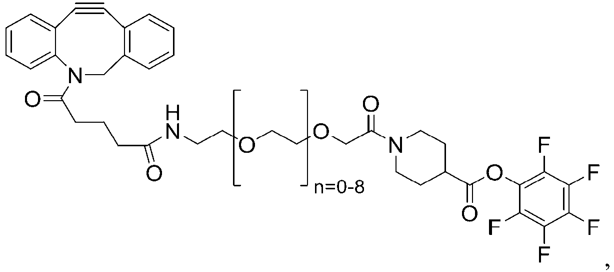 wherein n is an integer of 0-8.
17. The antibody-natural killer cell (NK cell) conjugate of embodiment 16, wherein the first linker is dibenzoazacyclooctyne-glutaryl-amino tetraethylene glycol-pentafluorophenyl acetylpiperidinate having the following structure: 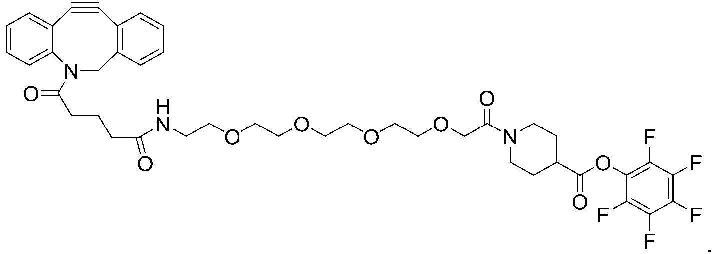
18. The antibody-natural killer cell (NK cell) conjugate of embodiment 13, wherein the second linker is an azidoacetylcyclohexosamine, e.g., an azidoacetylcyclogalactosamine or an azidoacetylglucosamine.
19. The antibody-natural killer cell (NK cell) conjugate of embodiment 18, wherein the second linker is 1,3,4,6-oxo-tetraacetyl-2-azidoacetamide-2-deoxy-a,b-D-galactose having the following structure: 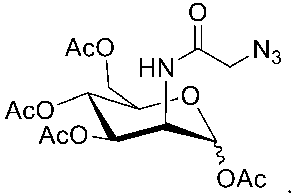
20. The antibody-natural killer cell (NK cell) conjugate of embodiment 19, wherein the second linker comprises at least 90% of the structure having a single α or β configuration.
21. A cell population comprising the antibody-natural killer cell (NK cell) conjugate of any one of embodiments 1-20.
22. The cell population of embodiment 21, wherein the count of CD3⁻ CD56⁺CD16⁺ cells is at least 95%, preferably at least 98% with respect to the total cell count in the cell population, and/or the count of CD3⁻CD56⁺NKG2D⁺ cells is at least 95%, preferably at least 98% with respect to the total cell count in the cell population.
23. The cell population of embodiment 21, wherein:
   the count of CD3⁺CD56⁺ cells is no more than 5% with respect to the total cell count in the cell population; and/or
   the count of CD3⁻CD19⁺ cells is no more than 2% with respect to the total cell count in the cell population; and/or
   the counts of CD3⁺CD4⁺ and CD3⁺CD8⁺ cells are no more than 2% with respect to the total cell count in the cell population.
24. The cell population of any one of embodiments 21-23, wherein, by cell count, the antibody-natural killer cell (NK cell) conjugate is at least 90%, preferably at least 95%, more preferably at least 98%, most preferably at least 99% with respect to the total cell count in the cell population.
25. A pharmaceutical composition comprising the antibody-natural killer cell (NK cell) conjugate of any one of embodiments 1-20 or the cell population of any one of embodiments 21-24, and a pharmaceutically acceptable carrier.
26. The pharmaceutical composition of embodiment 25, comprising sodium chloride and/or human serum albumin.
27. The pharmaceutical composition of embodiment 25 or 26, comprising trehalose, sucrose, dextran, DMSO, or any combination thereof.
28. The pharmaceutical composition of any one of embodiments 25-27, for use in the treatment of acute myeloid leukemia (AML) in an individual.
29. The pharmaceutical composition of embodiment 28, for use in the treatment of an initially diagnosed acute myeloid leukemia.
30. The pharmaceutical composition of embodiment 28, for use in the treatment of relapsed or refractory acute myeloid leukemia.
31. The pharmaceutical composition of embodiment 30, wherein the individual has received a chemotherapy and/or a targeted drug therapy.
32. The pharmaceutical composition of embodiment 31, wherein the targeted drug is gilteritinib and/or venentoclax.
33. The pharmaceutical composition of any one of embodiments 25-32, for use in combination with venentoclax and/or azacitidine for the treatment of relapsed or refractory acute myeloid leukemia.
34. Use of the antibody-natural killer cell (NK cell) conjugate of any one of embodiments 1-20 or the cell population of any one of embodiments 21-24 in the manufacture of a medicament for the treatment of acute myeloid leukemia (AML).
35. The use of embodiment 34, wherein the medicament is for the treatment of an initially diagnosed acute myeloid leukemia.
36. The use of embodiment 34, wherein the medicament is for the treatment of relapsed or refractory acute myeloid leukemia.
37. The use of embodiment 36, wherein the individual has received a chemotherapy and/or a targeted drug therapy.
38. The use of embodiment 37, wherein the targeted drug is gilteritinib and/or venentoclax.
39. The use of any one of embodiments 34-38, wherein the medicament is for use in combination with venentoclax and/or azacitidine for the treatment of relapsed or refractory acute myeloid leukemia.
40. A method of treating acute myeloid leukemia (AML) in an individual, the method comprising administering to the individual an effective amount of the antibody-natural killer cell (NK cell) conjugate of any one of embodiments 1-20 or the cell population of any one of embodiments 21-24 or the pharmaceutical composition of any one of embodiments 25-33.
41. The method of embodiment 40, wherein the individual suffers from an initially diagnosed acute myeloid leukemia.
42. The method of embodiment 40, wherein the individual suffers from relapsed or refractory acute myeloid leukemia.
43. The method of embodiment 42, wherein the individual has received a chemotherapy and/or a targeted drug therapy.
44. The method of embodiment 43, wherein the targeted drug is gilteritinib and/or venentoclax.
45. The method of any one of embodiments 40-44, further comprising administering to the individual venentoclax and/or azacitidine for combination treatment of relapsed or refractory acute myeloid leukemia.

### BRIEF DESCRIPTION OF THE DRAWINGS

FIG.1 shows a schematic diagram of the structure of an exemplary anti-CD33/CLL1 bispecific antibody-natural killer cell conjugate of the present application.
FIG.2 shows a schematic diagram of the mechanism of action of the exemplary anti-CD33/CLL1 bispecific antibody-natural killer cell conjugate of the present application.
FIG.3 shows the sequence and structural annotation of the exemplary anti-CD33/CLL1 bispecific antibody of the present application.
FIG.4 shows a schematic diagram of *in vitro* synthesis of the anti-CD33/CLL1 bispecific antibody according to an exemplary embodiment of the present application.
FIG.5 shows results of a flow cytometric analysis of IBR733 stock solution (Lot No. DSE20220407): CD56⁺, CD3⁻, CD16⁺, NKG2D⁺, CD19⁻.
FIG.6 shows results of a flow cytometric analysis of IBR733 formulation (Lot No. FE20220407): CD56⁺, CD3⁻, CD16⁺, NKG2D⁺, CD19⁻.
FIG.7 shows results of a flow cytometric analysis of UNK, IBR733 stock solution, and IBR733 formulation for the positivity rate and the geometric mean value of the conjugation.
FIG.8 shows a schematic diagram of a process for preparing L2 linker.
FIG.9 shows the structural formula of L2 linker, which is named as pentafluorophenyl (Z)-20-(1'-aza-2'-oxo-dibenzo[b,f]cyclo-7'-octynyl)-3,6,9,12-tetraoxa-15-aza-16,20-dioxodecanoyl piperidine-4-carboxylate.
FIG.10 shows a schematic diagram of a process for preparing N1 linker.
FIG.11 shows the structural formula of N1 linker, which is named as 1,3,4,6-tetra-O-acetyl-2-azidoacetylamido-2-deoxy-a,b-D-mannopyanose.
Figure 12 shows a schematic diagram of the conjugation mechanism between L2 linker and the bispecific antibody.
Figure 13 shows a schematic diagram of the conjugation mechanism between N1 linker and the NK cell.
FIG.14 shows a schematic diagram of the structure of an UNK attached with N1 linker.
FIG.15 shows a schematic diagram of the conjugation mechanism between the UNK and the L2 conjugated antibody.
FIG.16 shows a comparison of killing effects of IBR733, UNK cells, and NK cells on U937 cells.
FIG.17 shows a comparison of killing effects of IBR733, UNK cells, and NK cells on THP-1 cells.
FIG.18 shows a comparison of killing effects of IBR733, UNK cells, and NK cells on HL60 cells.
FIG.19 shows the levels of TraiL after IBR733 interacts with U937 tumor cells, with panel A and panel B showing the changes over time at an effector-to-target ratio of 10: 1, and panel C showing the changes over time at different effector-to-target ratios.
FIG.20 shows the levels of FasL after IBR733 interacts with U937 tumor cells, with panel A and panel B showing the changes over time at an effector-to-target ratio of 10: 1, and panel C showing the changes over time at different effector-to-target ratios.
Figure 21 shows survival curves for each group of animals during the experiment in a mouse model of U937 transplanted tumor. The ordinate shows the percentage of surviving animals and the abscissa shows the number of days.
Figure 22 shows survival curves for each group of animals during the experiment in a mouse model of THP-1 transplanted tumor. The ordinate shows the percentage of surviving animals and the abscissa shows the number of days.

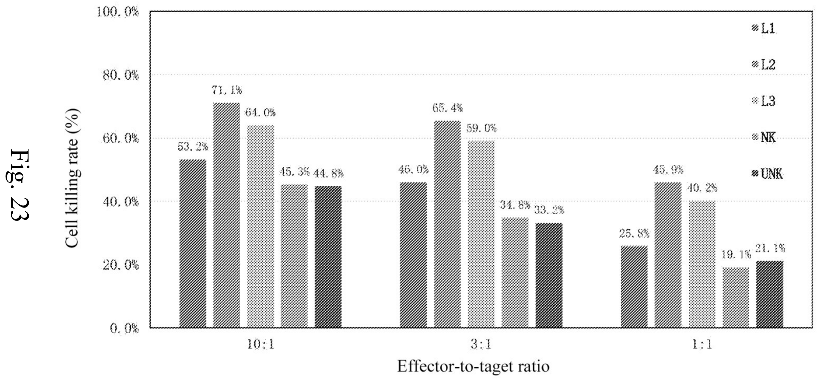

FIG.23 shows a comparison of killing effects of anti-CD33/CLL1 bispecific antibody-natural killer cell conjugates with different L linkers, UNK cells, and NK cells, on U937 cells, with the histograms for various effector-to-target ratio ordered from left to right as L1, L2, L3, NK, and UNK.

### DETAILED DESCRIPTION OF THE INVENTION

### Definition

Unless defined otherwise, all scientific and technical terms used herein have the same meaning as understood by a person of ordinary skill in the art. For definitions and terms in the art, a person skilled in the art can refer specifically to Current Protocols in Molecular Biology (Ausubel). Abbreviations for amino acid residues are the standard 3-letter and/or 1-letter codes used in the art to refer to one of the 20 common L-amino acids.

Although numerical ranges and parameter approximations are shown in broad ranges in the present application, the numerical values shown in the specific embodiments are described as accurately as possible. However, any numerical values inherently contain certain errors due to the standard deviation present in their respective measurements. Additionally, all ranges disclosed herein are to be understood as encompassing any and all subranges contained therein. For example, a range of "1 to 10" should be understood to encompass any and all subranges between the minimum value of 1 and the maximum value of 10, inclusive, i.e., all subranges starting with a minimum value of 1 or more, such as 1 to 6.1, and subranges ending with a maximum value of 10 or less, such as 5.5 to 10. Additionally, any reference referred to as "incorporated herein" should understood as being incorporated in its entirety.

As used herein, the term "individual" or "subject" refers to a mammal, such as a human, as well as other animals, such as wild animals, domestic animals, or experimental animals (e.g., gorillas, monkeys, rats, mice, rabbits, guinea pigs, woodchucks, or ground squirrels).

As used herein, the term "antigen" is a predetermined target to which an antibody can selectively bind. Examples of antigens include, but are not limited to, polypeptides, carbohydrates, nucleic acids, lipids, haptens, or other naturally occurring or synthetic compounds.

An "antibody", in its broad sense, refers to an immunoglobulin molecule that is capable of specifically binding to a target via at least one antigen recognition site located in the variable regions of the immunoglobulin molecule, and thus encompasses an intact antibody/full-length antibody, a single chain of an antibody, or any antigen binding fragment of an antibody (also referred to as an "antigen-binding portion"). When "antibody" and "antigen-binding fragment/antigen-binding portion" appear in the same context, an "antibody" can be understood as the entirety of the "antigen-binding fragment/antigen-binding portion", and they together correspond to the concept of an "antibody" in its broad sense.

The term "agonist antibody" is an antibody that initiates a reaction, for example, an antibody that mimics at least one functional activity of a target polypeptide. Agonist antibodies include antibodies that are ligand mimics, for example, wherein the ligand binds to a cell surface receptor, the binding induces cell signal transduction or activity via an intracellular cell signal transduction pathway, and wherein the antibody induces similar cell signal transduction or activation.

A "full-length/intact antibody" refers to a protein comprising at least two heavy (H) chains and two light (L) chains that are interconnected by disulfide bonds. Each heavy chain contains a heavy chain variable region (VH for short) and a heavy chain constant region. The heavy chain constant region contains three domains, CH1, CH2, and CH3. Each light chain contains a light chain variable region (VL for short) and a light chain constant region. The light chain constant region contains one domain, CL. The VH and VL regions can be further divided into a plurality of regions with high variability, referred to as complementarity determining regions (CDRs), interspersed with more conservative regions referred to as framework regions (FRs). Each VH or VL consists of three CDRs and four FRs, arranged from the amino terminus to the carboxy terminus in the following order: FR1, CDR1, FR2, CDR2, FR3, CDR3, and FR4. These variable regions of heavy and light chains contain binding domains that interact with antigens. The constant region of an antibody can mediate the binding of the immunoglobulin to tissues or factors in hosts, including various cells of the immune system (e.g., effector cells) and the first component of the classical complement system (Clq). A full-length/intact antibody can be any type of antibody, such as IgD, IgE, IgG, IgA or IgM (or subclasses of the above), but is not limited to any particular type. Immunoglobulins can be assigned to different classes depending on the amino acid sequences of the heavy chain constant domains of antibodies. Generally, immunoglobulins have five main classes, i.e., IgA, IgD, IgE, IgG and IgM. Several of these classes can be further classified into subclasses (isotypes), such as IgG1, IgG2, IgG3, IgG4, IgA1 and IgA2. Heavy chain constant domains corresponding to various immunoglobulin classes are referred to as α, δ, ε, γ and µ, respectively. Subunit structures and three-dimensional structures of the various immunoglobulin classes are well known. Chimeric or humanized antibodies are also encompassed by the antibodies according to the present application. It is well known to those skilled in the art that complementarity determining regions (CDRs, typically including CDR1, CDR2 and CDR3) are the subregions of the variable regions that have the most impact on the affinity and specificity of an antibody. CDR sequences in a VH or VL can be defined in multiple common ways, such as the Kabat definition, the IMGT definition, and the Chothia definition. For the variable region amino acid sequences of a given antibody, the CDR amino acid sequences in the VH and VL amino acid sequences can be determined according to different definitions. In embodiments of the present application, the CDR amino acid sequences are defined by the Kabat definition. For the variable region amino acid sequences of a given antibody, the CDR amino acid sequences in the variable region amino acid sequences can be analyzed in a variety of ways.

The term "humanized antibody" refers to an antibody obtained by grafting CDR sequences derived from another mammalian species, such as those in the mouse germline, onto human framework sequences. In order to retain binding affinity, some residues of the backbone segments (referred to as FR) can be modified. Humanized antibodies or fragments thereof according to the present application can be prepared by techniques known to those skilled in the art.

The term "chimeric antibody" refers to an antibody in which the variable region sequences are from one species and the constant region sequences are from another species, for example, an antibody in which the variable region sequences are from a murine antibody and the constant region sequences are from a human antibody. Chimeric antibodies or fragments thereof according to the present application can be prepared by using genetic recombination techniques. For example, a chimeric antibody can be produced by cloning a recombinant DNA comprising a promoter and sequences encoding variable regions of a non-human, particularly murine, monoclonal antibody according to the present application, and sequences encoding constant regions of a human antibody. Chimeric antibodies of the present application encoded by such recombinant genes will be, for example, a murine-human chimera, with its specificity determined by the variable regions derived from murine DNA and its isotype determined by the constant regions derived from human DNA.

The term "partially humanized antibody" refers to an antibody containing constant regions from a human and variable regions (including CDRs) from a non-human species (such as a mouse).

The term "semi-humanized antibody" is a type of humanized antibodies, and refers to an antibody in which one antibody chain comprises a murine variable region and the other antibody chain comprises a humanized variable region, i.e., a half-humanized antibody.

The term "monoclonal antibody" refers to an antibody obtained from a population of substantially homogeneous antibodies. That is, individual antibodies constituting the population are identical except that naturally occurring mutations may be present in a small number of individuals.

As used herein, the terms "antigen-binding fragment", "antigen-binding portion", and "antigen-binding region" are used interchangeably, and refer to parts of antibodies that contain amino acid residues interacting with antigens and conferring specificity and affinity to the binding agent, especially antibody fragments such as Fv, Fab, F(ab')₂, or Fab', or any fragment that can increase the half-life by chemical modification or by incorporation into liposomes, such as addition of poly(alkylene)glycols, such as polyethylene glycol ("pegylated") (referred to as pegylated fragments like Fv-PEG, scFv-PEG, Fab-PEG, F(ab')₂-PEG or Fab'-PEG) ("PEG" represents polyethylene glycol). Preferably, a functional fragment consists of or comprises a partial sequence of the heavy or light chain variable region of the antibody from which it is derived. The partial sequence is sufficient to retain the same binding specificity and sufficient affinity as the antibody from which it is derived. Such a functional fragment can comprise at least 5 amino acids, preferably 10, 15, 25, 50, and 100 contiguous amino acids of the antibody sequence from which it is derived. Examples of antigen-binding fragments include, but are not limited to, (1) Fab fragments, which can be monovalent fragments having VL-CL chains and VH-CH1 chains; (2) F(ab')₂ fragments, which can be divalent fragments having two Fab' fragments linked by disulfide bridges of the hinge region (i.e., dimers of Fab'); and (3) Fv fragments having VL and VH domains from a single arm of an antibody.

The term "single chain fragment variable (scFv)" refers to a single polypeptide chain formed by a VH domain and a VL domain that are linked via a peptide linker. A (scFv)₂ comprises two VH domains linked by a peptide linker and two VL domains that are combined with the two VH domains via disulfide bridges.

The term "Fc fragment", "Fc domain", "Fc moiety" or the like refers to a portion of the constant region of an antibody heavy chain, including the hinge region, the CH2 fragment and CH3 fragment of the constant region. The Fc region of the antibody may be subjected to engineering or modifications, including modifications related to effector functions, to such as reduce or eliminate antibody dependent cytotoxicity (ADCC) and/or complement-dependent cytotoxicity (CDC), which can be achieved by introducing one or more amino acid substitutions/mutations in the Fc region of the antibody.

The term "specific binding" refers to a non-random binding reaction between two molecules, such as the binding of an antibody to an antigenic epitope.

The term "bispecific antibody" refers to an antibody that has binding capacities for two antigenic epitopes. The two epitopes can be on different antigens or on the same antigen. Bispecific antibodies can adopt a variety of structural configurations. For example, a bispecific antibody can consist of two Fc fragments and two binding moieties fused to them, respectively (similar to a native antibody, except that the two arms bind to different antigenic targets or epitopes). An antigen binding moiety can be in the form of a single chain fragment variable (scfv) or a Fab fragment. The two different binding portions of the bispecific antibody each bind to the N-terminus of a single Fc fragment. The antigen-binding portions of the two arms may be configured in four combinations: scfv+Fab fragment, Fab fragment+scfv, scfv+scfv, and Fab fragment +Fab fragment. The Fc fragment may include mutations that ensure heavy chain heteropolymerization. The KIH (knob-in-hole) technique is a strategy for addressing heavy chain heteropolymerization. Generally, the KIH technique refers to modifying the amino acid sequence of the CH3 region to form a structure that facilitates pairing of heterologous incomplete antibodies, through which the normal antibody structure can be maintained as much as possible while constituting the bispecific antibody.

Generally, in order to prepare a monoclonal antibody or a functional fragment thereof, particularly a murine monoclonal antibody or a functional fragment thereof, one can refer to the techniques described in "Antibodies" manual (Harlow and Lane, Antibodies: A Laboratory Manual, Cold Spring Harbor Laboratory, Cold Spring Harbor NY, pp.726, 1988) or to the techniques for preparing a monoclonal antibody from hybridoma cells described by Kohler and Milstein (Nature, 256: 495-497, 1975).

The term "conservative variation" or "conservative amino acid substitutions" refers to substitutions that does not substantially affect or reduce the affinity of a protein. For example, an antibody can include up to about 1, up to about 2, up to about 5, up to about 10, or up to about 15 conservative substitutions, and specifically binds to the target antigen. The term "conservative variation" also includes the use of a substituted amino acid in place of the unsubstituted parent amino acid, as long as the antibody specifically binds to the target antigen.

The term "isolated" biological components (e.g., nucleic acids, proteins (including antibodies) or organelles) have been substantially isolated or purified from other biological components (i.e., other chromosomal and additional chromosomal DNA and RNA, proteins, and organelles) in the environment in which the components naturally occur (e.g., cells). Nucleic acids and proteins that have been "isolated" include those purified by standard purification methods. The term also includes nucleic acids and proteins that are prepared by recombinant expression in host cells, as well as chemically synthesized nucleic acids.

As used herein, the term "pharmaceutical composition" refers to a combination of at least one drug and, optionally, a pharmaceutically acceptable carrier or adjuvant that are combined together to achieve a particular purpose. In certain embodiments, the pharmaceutical composition includes a combination that is temporally and/or spatially separated, as long as it is capable of acting together to achieve the purpose of the present application. For example, the components contained in the pharmaceutical composition (e.g., antibody-cell conjugates according to the present application) can be administered to an individual together or separately. When the components contained in the pharmaceutical composition are separately administered to an individual, the components can be administered to the individual simultaneously or sequentially. The pharmaceutical composition according to the present application may include conventional components of a cell culture, particularly an NK cell culture, to maintain the activity of NK cells within the conjugate. The pharmaceutically acceptable carrier may include water, a buffered aqueous solution, an isotonic salt solution such as PBS (phosphate buffered saline), glucose, mannitol, dextrose, lactose, starch, magnesium stearate, cellulose, magnesium carbonate, 0.3% glycerol, hyaluronic acid, ethanol, or polyalkylene glycols such as polypropylene glycol, or triglycerides. The pharmaceutical composition or pharmaceutical preparation according to the present application may be administered via any suitable route, such as intravenous administration, intradermal, subcutaneous, intramuscular injection, or the like. The compositions according to the present application may comprise a wetting agent, an emulsifying agent, or a buffer substance as an additive.

As used herein, the term "a therapeutically effective amount" or "an effective amount" refers to a dose sufficient to show benefits to an individual being administered. The actual dose, rate, and timing of the administration will depend on the condition and severity of the disease in the individual to be treated. Prescription for the treatment (e.g., prescribed dose) is ultimately the responsibility of and dependent on the general practitioner or other physicians, and generally takes into account the disease being treated, the condition of individual patient, the delivery site, the administration method, and other factors known to the physicians.

The EC₅₀ value mainly refers to the concentration of a drug, antibody, toxin or the like that corresponds to 50% of the maximum biological effect after a specific period of exposure time. Pharmaceutically, in addition to being used to characterize the activation ability of agonists in *in vitro* experiments, it can also be used to denote the plasma concentration required to achieve half of the maximal biological effect *in vivo.* In some literature, EC₅₀ is also used to characterize the potency, including agonism and antagonism, of a compound at the cellular level. EC₅₀ values can be determined by methods such as ELISA.

The term "identity/homology/consistency" in the context of an amino acid or nucleic acid sequence is defined as the percentage of identical residues in an amino acid or nucleotide sequence variant that, after alignment and introduction of gaps, achieves maximum identity percentage, if desired. Methods and computer programs for sequence alignment are well known in the art.

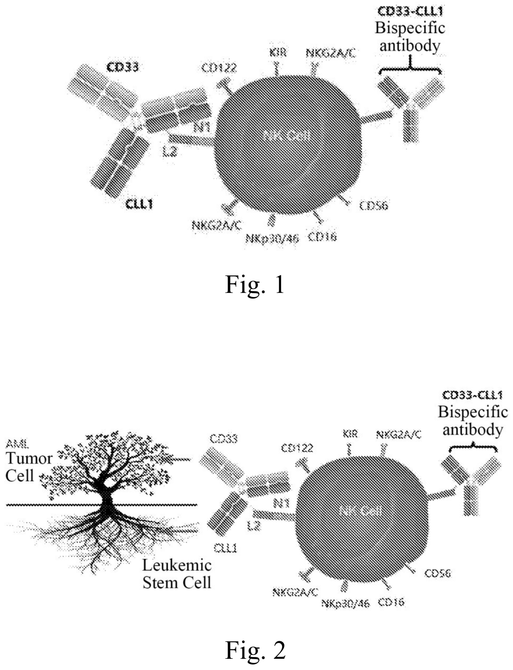

The inventors of the present application have conducted in-depth studies in the field of cellular immunotherapy. Particularly, an anti-CD33/CLL1 bispecific antibody-natural killer cell conjugate for acute myeloid leukemia (AML) has been developed. FIG.1 shows a schematic diagram of the structure of an exemplary anti-CD33/CLL1 bispecific antibody-natural killer cell conjugate of the present application. FIG.2 shows a schematic diagram of the mechanism of action of the exemplary anti-CD33/CLL1 bispecific antibody-natural killer cell conjugate of the present application.

NK cells recognize target antigens without major histocompatibility complex (MHC) restriction, and allogeneic NK cells carry a very low risk of graft-versus-host disease (GvHD). Therefore, allogeneic NK cell therapy is clinically feasible. In addition, NK cells have a very low risk of causing cytokine release syndrome (CRS). In a Phase I/II clinical trial of CAR-NK cell therapy for lymphoma published in the New England Journal of Medicine in 2020, Liu E et al. demonstrated that 8 out of the 11 enrolled subjects achieved remission after CAR-NK cell infusion, 7 of whom achieved complete remission, and none experienced CRS, neurotoxicity, or GvHD. It can be seen that CAR-NK cell therapy has a high safety. Moreover, it is based on the fact that NK cells recognize target antigens without MHC restriction, NK cells can be prepared into a universal product without being limited by autologous cells, allowing a wide range of NK cell sources for this therapy, such as allogeneic peripheral blood, umbilical cord blood, embryonic stem cells, human-induced pluripotent stem cells, and NK-92 cell lines.

The killing activity of NK cells is primarily mediated by:
1) direct lysis of target cells. NK cells release cytotoxic particles, such as perforin and granzyme, via exocytosis, activating the caspase pathway to induce necrosis or apoptosis of the target cells;
2) secretion of cytokines: cytokine-mediated killing by NK cells which are capable of synthesizing and secreting a variety of cytokines, such as IFN-γ, TNF-α, IL-1, IL-5, IL-8, IL-10, and G-CSF, to induce apoptosis of the target cells;
3) induction of apoptosis. activated NK cells express Fas (CD95) ligand and tumor necrosis factor-related apoptosis-inducing ligand (TRAIL) molecules, inducing apoptosis of CD95+ target cells and TRAIL receptor-positive target cells through a cascade reaction of endogenous enzymes;
4) ADCC: antibody-dependent cell-mediated cytotoxicity;
5) immune checkpoint pathways. NK cells express programmed death receptor 1 (PD-1), cytotoxic T-lymphocyte-associated protein 4 (CTLA4), and the like, and exert their effect by inhibiting the immune checkpoints.

The multiple mechanisms of action described above, as well as the potential and reliable safety of their use as universal products, make NK cell therapy an appealing immunotherapy.

NK cells currently used for treatment include natural NK cells, CAR-NK cells, and the like. In the field of natural NK cell therapy, CYNK-001 developed by Cellularity has acquired an FDA-granted Fast Track Designation for the treatment of acute myeloid leukemia. Similar to CAR-T cells, CAR-NK cells combine antigen-targeting capacity with natural killing capacity of NK cells, overcoming many limitations of other therapies for AML, such as severe myelosuppression due to chemotherapy, thereby creating a new platform for cytopharmaceuticals. In addition, natural receptors of CAR-NK cells, such as NKp46, NKp30, NKp44, NKG2D and CD226, are retained, which can independently recognize their ligands and retain the basic killing function of NK cells.

In the present application, one of the targets selected for the bispecific antibody is CD33, which is expressed in more than 90% of the blast cells in patients with acute myeloid leukemia, but is not expressed on the surface of hematopoietic stem cells nor in mature granulocytes and other tissues. Therefore, CD33 is a good target for the treatment of myeloid leukemia.

In the present application, another target selected for the bispecific antibody is C-type lectin-like molecule 1 (CLL-1), which present in myeloid cells in peripheral blood and bone marrow, and in most AML leukemic cells, and is expressed in most CD34⁺CD38⁻ stem cells of AML patients, but not in CD34⁺CD38⁻ stem cells of a normal human. CLL1 receptor targets leukemic stem cells (a small subset of slowly growing leukemic cells that are resistant to conventional drug therapies and can stably proliferate into new leukemic cell branches), and CLL1 is associated with leukemic stem cells and disease recurrence. CLL1 as a marker antigen for leukemic stem cells contributes to the distinguishing between normal stem cells and leukemic stem cells, and contributes to a specific targeted cell immunotherapy of AML with CLL1 as the target.

In a first aspect, the present application provides an antibody-natural killer cell (NK cell) conjugate, wherein the antibody is a bispecific antibody comprising a CD33 antigen-binding fragment and a CLL1 antigen-binding fragment, and the bispecific antibody is conjugated to the NK cell via a linker.

In some embodiments, the CD33 antigen-binding fragment comprises:
HCDR1 as set forth in SEQ ID NO:9 or having at least 80%, 85%, 90%, 95% or 99% identity to the sequence set forth in SEQ ID NO:9,
HCDR2 as set forth in SEQ ID NO: 10 or having at least 80%, 85%, 90%, 95% or 99% identity to the sequence set forth in SEQ ID NO:10,
HCDR3 as set forth in SEQ ID NO:11 or having at least 80%, 85%, 90%, 95% or 99% identity to the sequence set forth in SEQ ID NO:11,
LCDR1 as set forth in SEQ ID NO: 12 or having at least 80%, 85%, 90%, 95% or 99% identity to the sequence set forth in SEQ ID NO:12,
LCDR2 as set forth in SEQ ID NO:13 or having at least 80%, 85%, 90%, 95% or 99% identity to the sequence set forth in SEQ ID NO:13, and
LCDR3 as set forth in SEQ ID NO: 14 or having at least 80%, 85%, 90%, 95% or 99% identity to the sequence set forth in SEQ ID NO:14;
wherein the amino acid sequences of the HCDRs and the LCDRs are defined according to Kabat.

In some embodiments, the amino acid sequence of the heavy chain variable region of the CD33 antigen-binding fragment is set forth in SEQ ID NO:5 or has at least 80%, 81%, 82%, 83%, 84%, 85%, 86%, 87%, 88%, 89%, 90%, 91%, 92%, 93%, 94%, 95%, 96%, 97%, 98% or 99% identity to the sequence set forth in SEQ ID NO:5, and the amino acid sequence of the light chain variable region is set forth in SEQ ID NO:7 or has at least 80%, 81%, 82%, 83%, 84%, 85%, 86%, 87%, 88%, 89%, 90%, 91%, 92%, 93%, 94%, 95%, 96%, 97%, 98% or 99% identity to the sequence set forth in SEQ ID NO:7.

In some embodiments, the amino acid sequence of the heavy chain variable region of the CD33 antigen binding fragment differs from the amino acid sequence set forth in SEQ ID NO: 5 by about 1, 2, 3, 4, 5, 6, 7, 8, 9, or 10 amino acid substitutions, deletions, and/or additions. In some embodiments, the C-terminal or N-terminal region of the amino acid sequence set forth in SEQ ID NO: 5 can be truncated by about 1, 2, 3, 4, 5, 6, 7, 8, 9, 10, 11, 12, 13, 14, 15, 20, 25, or more amino acids, while still retaining a function similar to that of the heavy chain variable region of the antibody. In some embodiments, the C-terminal or N-terminal region of the amino acid sequence set forth in SEQ ID NO: 5 is added with 1, 2, 3, 4, 5, 6, 7, 8, 9, 10, 11, 12, 13, 14, 15, 20, 25 or more amino acids, and the resulting amino acid sequence still retains a function similar to that of the heavy chain variable region of the antibody. In some embodiments, 1, 2, 3, 4, 5, 6, 7, 8, 9, 10, 11, 12, 13, 14, 15, 20, 25 or more amino acids may be added to or deleted from a region other than the C-terminal or the N-terminal of the amino acid sequence set forth in SEQ ID NO: 5, as long as the altered amino acid sequence substantially retains a function similar to that of the heavy chain variable region of the antibody.

In some embodiments, the amino acid sequence of the light chain variable region of the CD33 antigen binding fragment differs from the amino acid sequence set forth in SEQ ID NO: 7 by about 1, 2, 3, 4, 5, 6, 7, 8, 9, or 10 amino acid substitutions, deletions, and/or additions. In some embodiments, the C-terminal or N-terminal region of the amino acid sequence set forth in SEQ ID NO: 7 may also be truncated by about 1, 2, 3, 4, 5, 6, 7, 8, 9, 10, 11, 12, 13, 14, 15, 20, 25, or more amino acids, while still retaining a function similar to that of the light chain variable region of the antibody. In some embodiments, 1, 2, 3, 4, 5, 6, 7, 8, 9, 10, 11, 12, 13, 14, 15, 20, 25 or more amino acids may also be added to the C-terminal or N-terminal region of the amino acid sequence set forth in SEQ ID NO: 7, and the resulting amino acid sequence still retains a function similar to that of the light chain variable region of the antibody. In some embodiments, 1, 2, 3, 4, 5, 6, 7, 8, 9, 10, 11, 12, 13, 14, 15, 20, 25 or more amino acids may also be added to or deleted from a region other than the C-terminal or the N-terminal of the amino acid sequence set forth in SEQ ID NO: 7, as long as the altered amino acid sequence substantially retains a function similar to that of the light chain variable region of the antibody.

In some embodiments, the CLL1 antigen-binding fragment comprises:
HCDR1 as set forth in SEQ ID NO: 15 or having at least 80%, 85%, 90%, 95% or 99% identity to the sequence set forth in SEQ ID NO:15,
HCDR2 as set forth in SEQ ID NO: 16 or having at least 80%, 85%, 90%, 95% or 99% identity to the sequence set forth in SEQ ID NO:16,
HCDR3 as set forth in SEQ ID NO:17 or having at least 80%, 85%, 90%, 95% or 99% identity to the sequence set forth in SEQ ID NO:17,
LCDR1 as set forth in SEQ ID NO:18 or having at least 80%, 85%, 90%, 95% or 99% identity to the sequence set forth in SEQ ID NO:18,
LCDR2 as set forth in SEQ ID NO:19 or having at least 80%, 85%, 90%, 95% or 99% identity to the sequence set forth in SEQ ID NO:19, and
LCDR3 as set forth in SEQ ID NO:20 or having at least 80%, 85%, 90%, 95% or 99% identity to the sequence set forth in SEQ ID NO:20;
wherein the amino acid sequences of the HCDRs and the LCDRs are defined according to Kabat.

In some embodiments, the amino acid sequence of the heavy chain variable region of the CLL1 antigen-binding fragment is set forth in SEQ ID NO:1 or has at least 80%, 81%, 82%, 83%, 84%, 85%, 86%, 87%, 88%, 89%, 90%, 91%, 92%, 93%, 94%, 95%, 96%, 97%, 98% or 99% identity to the sequence set forth in SEQ ID NO:1, and the amino acid sequence of the light chain variable region is set forth in SEQ ID NO:3 or has at least 80%, 81%, 82%, 83%, 84%, 85%, 86%, 87%, 88%, 89%, 90%, 91%, 92%, 93%, 94%, 95%, 96%, 97%, 98% or 99% identity to the sequence set forth in SEQ ID NO:3.

In some embodiments, the amino acid sequence of the heavy chain variable region of the CLL1 antigen binding fragment differs from the amino acid sequence set forth in SEQ ID NO: 1 by about 1, 2, 3, 4, 5, 6, 7, 8, 9, or 10 amino acid substitutions, deletions, and/or additions. In some embodiments, the C-terminal or N-terminal region of the amino acid sequence set forth in SEQ ID NO: 1 can be truncated by about 1, 2, 3, 4, 5, 6, 7, 8, 9, 10, 11, 12, 13, 14, 15, 20, 25, or more amino acids, while still retaining a function similar to that of the heavy chain variable region of the antibody. In some embodiments, the C-terminal or N-terminal region of the amino acid sequence set forth in SEQ ID NO: 1 is added with 1, 2, 3, 4, 5, 6, 7, 8, 9, 10, 11, 12, 13, 14, 15, 20, 25 or more amino acids, and the resulting amino acid sequence still retains a function similar to that of the heavy chain variable region of the antibody. In some embodiments, 1, 2, 3, 4, 5, 6, 7, 8, 9, 10, 11, 12, 13, 14, 15, 20, 25 or more amino acids may be added to or deleted from a region other than the C-terminal or the N-terminal of the amino acid sequence set forth in SEQ ID NO: 1, as long as the altered amino acid sequence substantially retains a function similar to that of the heavy chain variable region of the antibody.

In some embodiments, the amino acid sequence of the light chain variable region of the CLL1 antigen binding fragment differs from the amino acid sequence set forth in SEQ ID NO: 3 by about 1, 2, 3, 4, 5, 6, 7, 8, 9, or 10 amino acid substitutions, deletions, and/or additions. In some embodiments, the C-terminal or N-terminal region of the amino acid sequence set forth in SEQ ID NO: 3 may also be truncated by about 1, 2, 3, 4, 5, 6, 7, 8, 9, 10, 11, 12, 13, 14, 15, 20, 25, or more amino acids, while still retaining a function similar to that of the light chain variable region of the antibody. In some embodiments, 1, 2, 3, 4, 5, 6, 7, 8, 9, 10, 11, 12, 13, 14, 15, 20, 25 or more amino acids may also be added to the C-terminal or N-terminal region of the amino acid sequence set forth in SEQ ID NO: 3, and the resulting amino acid sequence still retains a function similar to that of the light chain variable region of the antibody. In some embodiments, 1, 2, 3, 4, 5, 6, 7, 8, 9, 10, 11, 12, 13, 14, 15, 20, 25 or more amino acids may also be added to or deleted from a region other than the C-terminal or the N-terminal of the amino acid sequence set forth in SEQ ID NO: 3, as long as the altered amino acid sequence substantially retains a function similar to that of the light chain variable region of the antibody.

In some embodiments, the forms of the CD33 antigen binding fragment and the CLL1 antigen binding fragment are independently selected from a single chain antibody (scFv) or a Fab fragment.

In some embodiments, the bispecific antibody comprises a CD33 antibody arm comprising the CD33 antigen-binding fragment, and a CLL1 antibody arm comprising the CLL1 antigen-binding fragment, wherein
the CD33 antibody arm comprises a heavy chain variable region set forth in SEQ ID NO:5, a heavy chain constant region set forth in SEQ ID NO:6, a light chain variable region set forth in SEQ ID NO:7, and a light chain constant region set forth in SEQ ID NO:8; and/or
the CLL1 antibody arm comprises a heavy chain variable region set forth in SEQ ID NO: 1, a heavy chain constant region set forth in SEQ ID NO:2, a light chain variable region set forth in SEQ ID NO:3, and a light chain constant region set forth in SEQ ID NO:4.

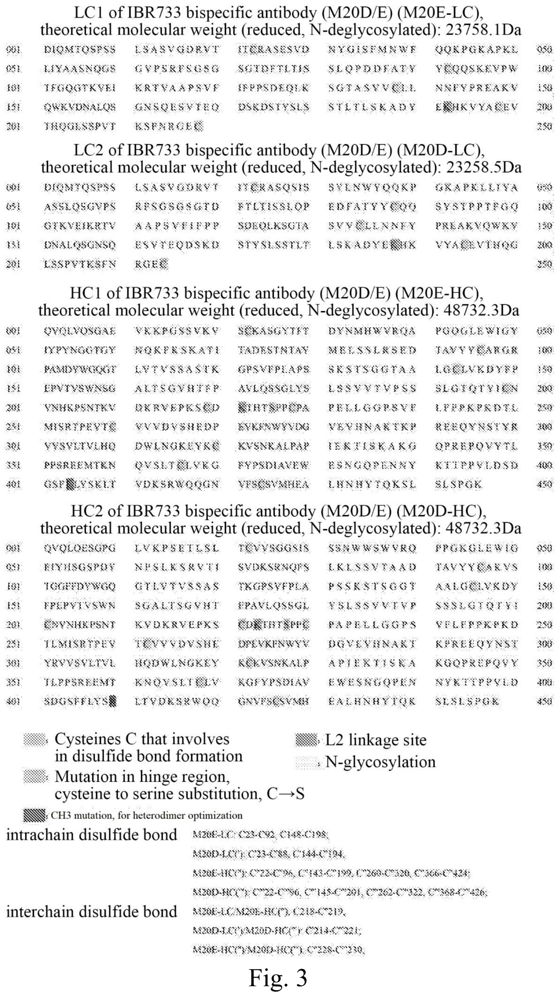

FIG.3 shows the sequence and structural annotation of an exemplary anti-CD33/CLL1 bispecific antibody comprising the CD33 antibody arm and the CLL1 antibody arm described above.

In some embodiments, the bispecific antibody is an intact antibody.

In some embodiments, the bispecific antibody is a humanized antibody or a fully human antibody.

In some embodiments, the bispecific antibody is a monoclonal antibody.

In some embodiments, the bispecific antibody is of IgG1, IgG2 or IgG4 isotype. In some embodiments, the bispecific antibody is of IgG1 isotype.

In some embodiments, the bispecific antibody comprises a light chain constant region of κ subtype.

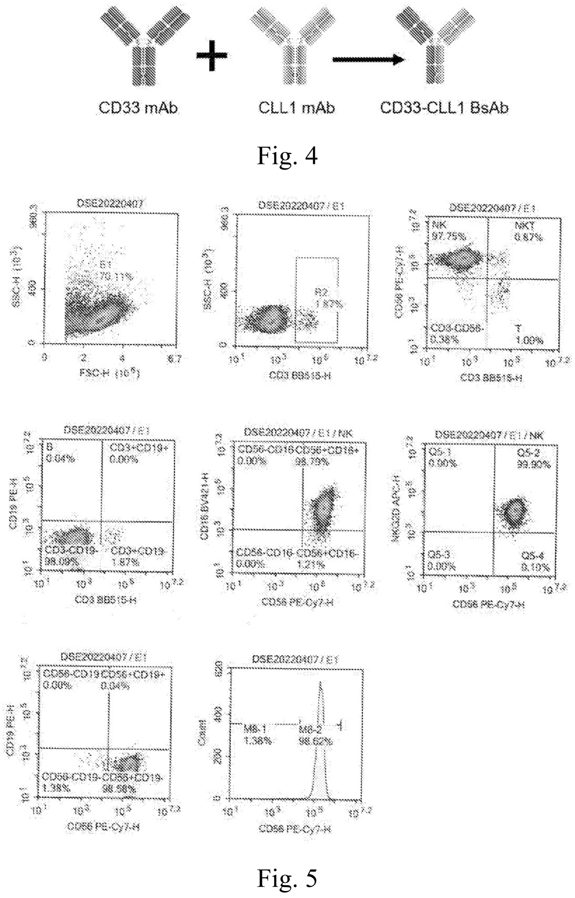

In the case of a given antibody structure/sequence, techniques for preparing the corresponding bispecific antibody are well known to those skilled in the art. An exemplary methodology for preparing the anti-CD33/CLL1 bispecific antibody in the examples of the present application is to first prepare anti-CD33 and anti-CLL1 monoclonal antibodies, and then reduce the disulfide bond in the hinge region such that the arms of the monoclonal antibodies are separated and reassembled into the bispecific antibody (see FIG.4 for a schematic diagram of the reaction).

Specifically, the sequences of the anti-CD33 and anti-CLL1 monoclonal antibodies were obtained from phage library screening. The anti-CD33/CLL1 antibody is obtained by screening for the binding activity between the target proteins and cells expressing the target proteins, followed by confirmation through affinity studies. The anti-CLL1 monoclonal antibody includes the heavy chain variable region set forth in SEQ ID NO:1 (HCDR1-3 being SEQ ID NO: 15, 16, 17, respectively), the heavy chain constant region set forth in SEQ ID NO:2, the light chain variable region set forth in SEQ ID NO:3 (LCDR1-3 being SEQ ID NO:18, 19, 20, respectively), and the light chain constant region set forth in SEQ ID NO:4. The anti-CD33 monoclonal antibody includes the heavy chain variable region set forth in SEQ ID NO:5 (HCDR1-3 being SEQ ID NO:9, 10, 11, respectively), the heavy chain constant region set forth in SEQ ID NO:6, the light chain variable region set forth in SEQ ID NO:7 (LCDR1-3 being SEQ ID NO:12, 13, 14, respectively), and the light chain constant region set forth in SEQ ID NO:8. The DNA sequences of the anti-CD33 and anti-CLL1 antibodies are determined, and recombinant plasmids expressing either the anti-CD33 monoclonal antibody or the anti-CLL1 monoclonal antibody, designated as IB12-CD33 and IB12-CLL1 in the examples, are constructed. The construction of monoclonal cell strains is based on known techniques, where the IB12-CD33 and IB12-CLL1 plasmids are electrotransfected into CHO cells, subjected to one round of minipool screening, followed by two rounds of screening after monoclones are plated, and confirmed by primary culture for passage stability, resulting in a cell strain stably expressing the anti-CLL1 monoclonal antibody and a cell strain stably expressing the anti-CD33 monoclonal antibody. In the preparation of the anti-CD33-CLL1 bispecific antibody, the above-mentioned cell strains stably expressing the anti-CLL1 monoclonal antibody and the anti-CD33 monoclonal antibody are expanded, subjected to a mass culture, and collected for the unprocessed bulk (UPB), which undergoes clarification and filtration to generate the cell culture supernatants. The supernatants contain the anti-CD33 monoclonal antibody and the anti-CLL1 monoclonal antibody, respectively. Then, the anti-CD33 monoclonal antibody and the anti-CLL1 monoclonal antibody are subjected to a protein purification process via Protein A affinity purification, then assembled into the anti-CD33-CLL1 bispecific antibody under the conditions of reducing agent 2-MEA and air oxidation, and then subjected to a two-step chromatography of anion exchange and cation exchange to obtain the anti-CD33-CLL1 bispecific antibody.

In addition to the methodology described above, a plasmid (e.g., a three-plasmid system or a four-plasmid system) expressing the antigen-binding portions (Fab or scFv) and the constant regions of the bispecific antibody can also be transfected into a cell, and the expressed antibody fragments are directly assembled into the bispecific antibody. The "Knob-into-hole" and "CrossMab" techniques can be used in such method.

In some embodiments, the NK cell is CD16⁺ and/or NKG2D⁺. In some embodiments, the NK cell is CD16⁺NKG2D⁺.

In some embodiments, the conjugate comprises at least 90%, e.g., at least 95%, at least 98%, or at least 99% of CD16⁺NKG2D⁺ NK cells. In some embodiments, the conjugate comprises at least 90% of CD16⁺NKG2D⁺ NK cells, and at least 95% (e.g., at least 96%, at least 97%, at least 98%, or at least 99%) of CD56⁺ NK cells.

There are a variety of techniques for *in vitro* culturing, expanding, and obtaining NK cells, and their general principles and methodologies are known to those skilled in the art.

In some embodiments, NK cells are obtained through *in vitro* culture and expansion of NK cells derived from peripheral blood mononuclear cells (PBMCs), which is also an exemplary method in the examples of the present application. PBMC is one of the main sources of NK cells, offering advantages such as relatively easy collection, ease of *in vitro* expansion, and the absence of toxic and side effects. However, the proportion of NK cells in PBMCs is only 10%-15%, and methods for expanding PBMC-derived NK cells include stimulating their *in vitro* expansion with a combination of cytokines, feeder cells, or membrane particles. These different systems for expansion show varying levels of NK cell expansion efficiency. In some embodiments, HLA and KIR of PBMCs are screened. In some embodiments, variants of CD16a in PBMCs, i.e., 176V, 176F, are screened. In some embodiments, the activity of the natural killer cells during culture is maintained or activated with one or more cytokines. In some embodiments, the proliferation of B cells, macrophages, and other immune cells is inhibited with one or more immunoglobulins or fusion proteins.

PBMCs may be derived from apheresis of peripheral blood lymphocytes from an allogeneic healthy donor. After separating from T cells and red blood cells, the cells are transferred to a primary cell cryopreservation solution and aliquoted according to the specification of ≥ 6.0×10⁷ viable cells per vial to obtain PBMCs. PBMCs are stored at ≤ -175°C in liquid nitrogen for long-term storage. PBMCs frozen in liquid nitrogen are removed, resuscitated in a suitable medium, cultured in an enlarged culture scale (i.e., expanded in an increased volume of culture medium) and added with NK cell-related cytokines which include but are not limited to IL2 and IL15, to maintain their proliferation capacity and activity. Exemplary preparation method can be found in Example 1 of the present application.

In some embodiments, NK cells are obtained through *in vitro* culture and expansion of NK cells derived from umbilical cord blood. There are generally two different methods for obtaining a substantial amount of NK cells from the umbilical cord blood. One method is to expand NK cells in the umbilical cord blood, while the other method is to induce CD34⁺ hematopoietic stem/progenitor cells in the umbilical cord blood to differentiate into NK cells and then expand them.

In some embodiments, NK cells are obtained from *in vitro* culture and expansion of an NK cell line. As an example, NK-92 as a homogeneous immortalized NK lymphoma cell is the first NK cell-based immunotherapy approved by the FDA for clinical trials.

In some embodiments, NK cells are obtained through *in vitro* induction, culture, and expansion of induced pluripotent stem cells (iPSCs) or mesenchymal stem cells (ESCs).

In some embodiments, the bispecific antibody is conjugated to the NK cell via click chemistry of linkers.

Click chemistry, also known as "link chemistry" or "speed matching combined chemistry", is a concept of synthesis that was introduced by chemist Barry Sharpless in 2001. The main idea is to fast and reliably complete chemical synthesis of various molecules through the splicing of small units. There are four main types of click chemistry reactions: cycloaddition reaction, nucleophilic ring-opening reaction, non-aldol type carbonyl reaction, and carbon-carbon multiple bond addition reaction.

In some embodiments, the bispecific antibody is conjugated to the NK cell via a first linker conjugated to the bispecific antibody, and a second linker conjugated to the NK cell, with the first and second linkers conjugated to each other to form the antibody-NK cell conjugate.

In some embodiments, the first linker is an active ester capable of forming a conjugation to a lysine residue of the antibody by a reaction converting an ester bond into an amide bond, for example, the active ester is a pentafluorophenyl ester, such as pentafluorophenyl piperidinate. The active ester can be a molecule that is stable in an aqueous phase and can specifically and covalently bind to the lysine residue of the antibody. In some embodiments, the active ester contains a tetraethylene glycol chain structure that undergoes slow hydrolysis in water. In some embodiments, the reactive ester reacts with an amino group at the hydrophilic interface of the protein, converting the ester bond into an amide bond, thereby establishing a conjugation mechanism.

In some embodiments, the first linker further comprises a carbon-carbon triple bond structure capable of undergoing a cyclization reaction with an azide group to form a five-membered ring of triazole, for example, the carbon-carbon triple bond structure is an octyne group.

In some embodiments, the first linker is a dibenzoazacyclooctyne-glutaryl-amino polyethylene glycol-pentafluorophenyl acetylpiperidinate having the following structure: 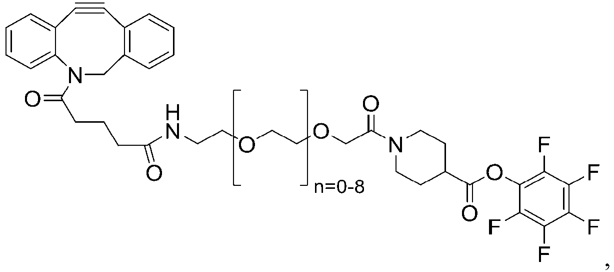 wherein n is an integer of 0-8.

In some embodiments, the first linker is dibenzoazacyclooctyne-glutaryl-amino tetraethylene glycol-pentafluorophenyl acetylpiperidinate having the following structure:

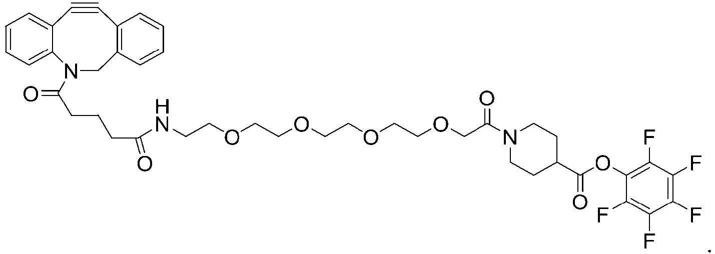

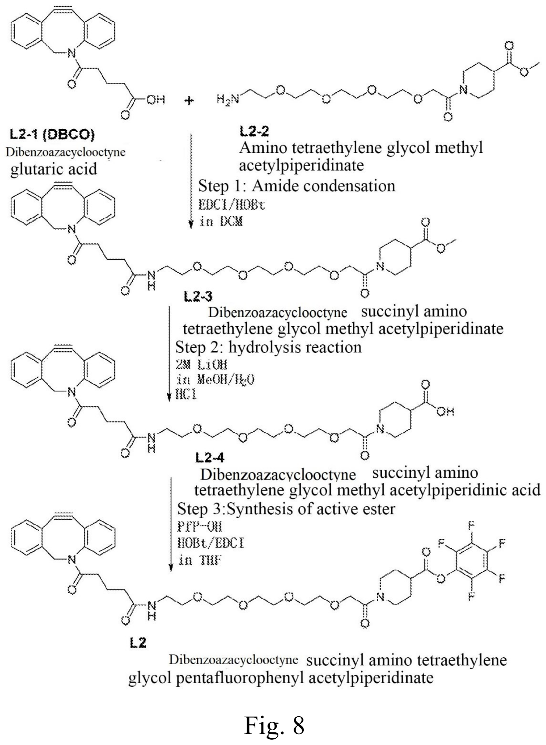

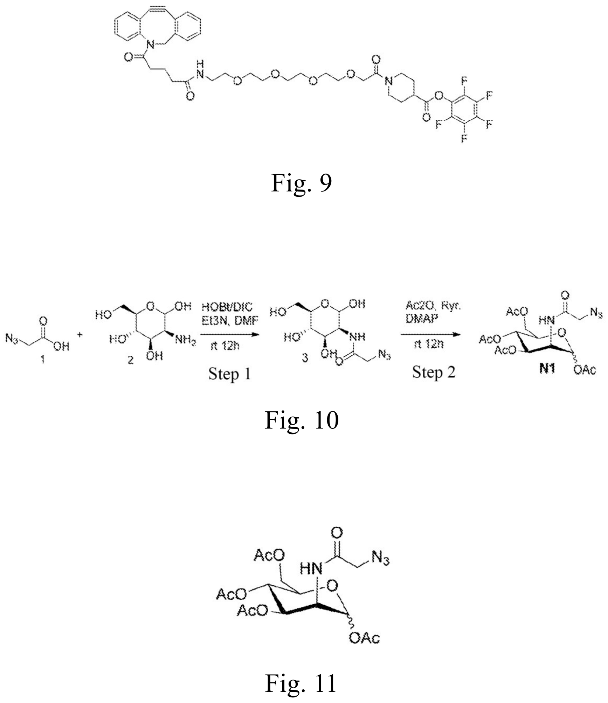

Dibenzoazacyclooctyne-glutaryl-amino tetraethylene glycol-pentafluorophenyl acetylpiperidinate is also referred to as L2 linker in the present application. A process for synthesizing L2 linker is shown in Figure 8, and the structural formula of L2 linker is shown in Figure 9. The process includes three main synthetic steps: amide condensation, hydrolysis reaction, and synthesis of active ester.

### Step 1 - Amide condensation

Dibenzoazacyclooctyne glutaric acid (L2-1) and amino tetraethylene glycol methyl acetylpiperidinate (L2-2) are commercially available. An intermediate dibenzoazacyclooctyne glutaryl amino tetraethylene glycol methyl acetylpiperidinate (L2-3) is obtained according to the chemical condensation reaction. L2-3 is a stable compound.

Specifically, compound L2-1 and compound L2-2 (1:1.1, with L2-2 in excess) are dissolved in a dichloromethane (DCM) solution. Hydroxybenzotriazole (HOBt) and 1-ethyl-(3-dimethylaminopropyl)carbodiimide (EDCI) are separately added, followed by the addition of triethanolamine (TEA). The mixture is stirred at room temperature for 4-12 hours, quenched with water, extracted twice with dichloromethane (DCM), and purified with a silica gel column (dichloromethane: methanol = 20:1) to yield compound L2-3 as a yellow oil.

### Step 2 - Hydrolysis reaction

Intermediate L2-3 obtained in Step 1 is hydrolyzed to yield an intermediate dibenzoazacyclooctyne glutaryl amino tetraethylene glycol acetylpiperidinic acid (L2-4). The hydrolysis reaction is typically a quantitative reaction, and the product is used directly in the next synthesis step without further purification.

Specifically, compound L2-3 is dissolved in a mixed solution of methanol and water, cooled to 0°C, and then added with an aqueous solution of 1 mol/L lithium hydroxide (LiOH). The mixture is stirred for 4-12 hours as the temperature increases from 0°C to room temperature, acidified to pH between 2 and 3 with 1 mol/L HCl, extracted three times with ethyl acetate (EA) and dried to yield the intermediate dibenzoazacyclooctyne glutaryl amino tetraethylene glycol acetylpiperidinic acid (L2-4) as a yellow oil.

### Step 3 - Synthesis of active ester

Intermediate L2-4 obtained in Step 2 (without purification) is condensed with pentafluorophenol and dicyclohexylcarbodiimide (DCC) to yield L2, i.e., dibenzoazacyclooctyne-glutaryl-amino tetraethylene glycol-pentafluorophenyl acetylpiperidinate.

Specifically, compound L2-4 is dissolved in tetrahydrofuran (THF), cooled to 0°C, and then added with pentafluorophenol, hydroxybenzotriazole (HOBt), and dicyclohexylcarbodiimide (DCC). The mixture is stirred for 4-12 hours with temperature increasing from 0°C to room temperature, extracted three times with ethyl acetate (EA), dried and purified with a silica gel column (dichloromethane: methanol = 20:1) to yield dibenzoazacyclooctyne-glutaryl-amino tetraethylene glycol-pentafluorophenyl acetylpiperidinate (L2) as a yellow oil, the structural formula of which is shown in FIG. 9.

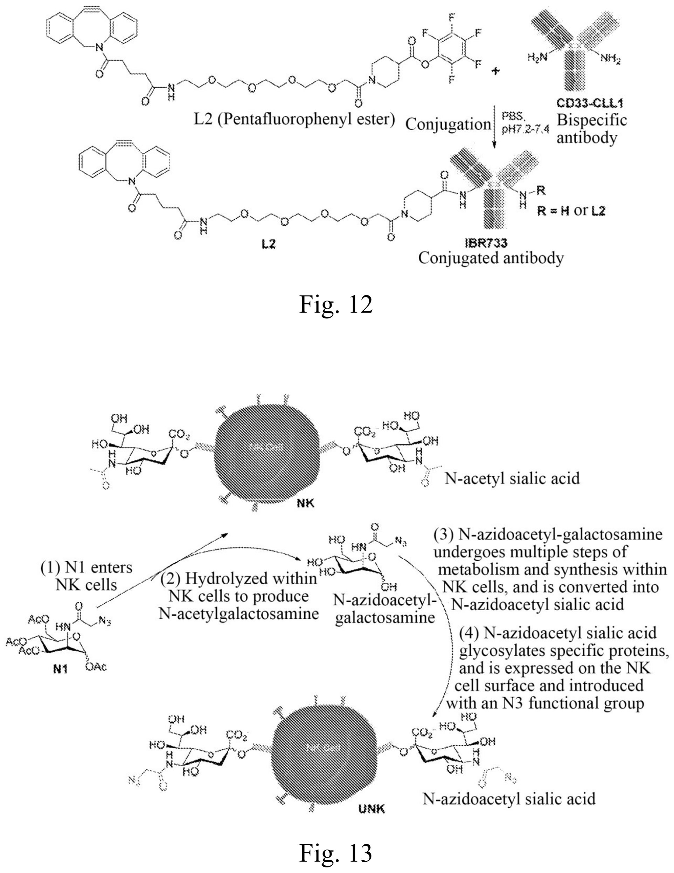

Subsequently, L2 linker may be subjected to a site-directed coupling reaction with the anti-CD33-CLL1 bispecific antibody in a system of PBS at pH 7.2-7.4, preferably HEPES at pH 7.2 (see FIG. 12 for a schematic illustration). Typically, one anti-CD33-CLL1 bispecific antibody can be conjugated to 1-4 (preferably 1-2) L2 linkers. The reaction can be terminated by adjusting the pH to about 5.0 to yield a L2-conjugated antibody.

In some embodiments, the second linker is azidoacetylcyclohexosamine, such as azidoacetylcyclogalactosamine or azidoacetylglucosamine. In some embodiments, the second linker is a molecule that is stable in an aqueous phase and can specifically and covalently bind to a membrane protein modified by sialic acid. In some embodiments, the second linker is transferred to the membrane protein modified by sialic acid via metabolic pathways in the cell itself during the cell culture. In some embodiments, the azidoacetyl group of the second linker can undergo a cyclization reaction with the carbon-carbon triple bond of the first linker to form a stable five-membered ring of triazole.

In some embodiments, the second linker is 1,3,4,6-oxo-tetraacetyl-2-azidoacetamide-2-deoxy-a,b-D-galactose having the following structure:

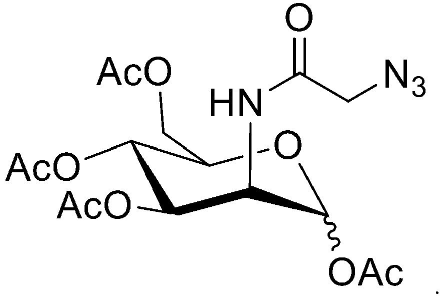

In some embodiments, the second linker comprises at least 90%, for example, at least 95%, at least 98%, or at least 99% of the structure having a single α or β configuration.

1,3,4,6-oxo-tetraacetyl-2-azidoacetamide-2-deoxy-a,b-D-galactose is also referred to as N1 linker in the present application. A process for synthesizing N1 linker is shown in FIG. 10, and the structural formula of N1 linker is shown in FIG. 11. The process includes two main synthetic steps: amino azidoacetylation and hydroxyacetylation.

### Step 1 - Amino azidoacetylation

1.2 equivalents of a-azidoacetic acid (compound 1), 2 equivalents of hydroxybenzotriazole (HOBt), and triethylamine (Et₃N) are added to a solution of D-galactosamine hydrochloride (compound 2) in N,N-dimethylformamide (DMF). A small amount of methanol (MeOH) is added to assist dissolution. The reaction is performed at room temperature for 12 hours. The reaction mixture is poured into dichloromethane (DCM)/methanol (MeOH) and mixed by shaking. An ether is added to precipitate the oily product (compound 3), and the ether layer is decanted. This process is repeated twice, followed by vacuum drying. The product is used in the next reaction without further purification.

### Step 2 - Hydroxyacetylation

Compound 3 obtained above is added to anhydrous pyridine (Pyr.) and acetic anhydride (Ac₂O), followed by the addition of 4-dimethylaminopyridine (DMAP) catalyst at room temperature. The reaction is allowed to proceed for 12 hours. When the reaction is nearly complete, as detected by HPLC, the mixture is concentrated to obtain a racemic mixture of tetraacetyl-N-azidoacetyl-a,b-D-galactosamine. Ethyl acetate/petroleum ether is used to precipitate the product as a solid, mainly in a b-configuration, with a purity of > 85%. Further purification is performed with a silica gel column (dichloromethane: methanol = 20:1), resulting in the N1 product with a single optical isomer purity greater than 90%. Two a,b-isomers can be isomerized to each other in cells, converted to N-azidoacetyl sialic acid through multiple steps of metabolism andsynthesis, and finally expressed on glycoproteins of the NK cell surface. The structural formula of N1 linker is shown in FIG. 11.

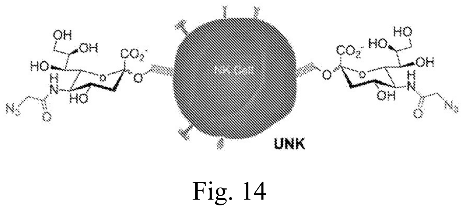

Once NK cells are cultured for 15-16 days, the culture medium is added with N1 linker and incubated for 12-18 hours to obtain N1 linker-modified NK cells (referred to as "UNK" in the examples of the present application) (see Figures 13 and 14 for a schematic diagram of the conjugation mechanism of N1 linker to NK cells and the structure of UNK).

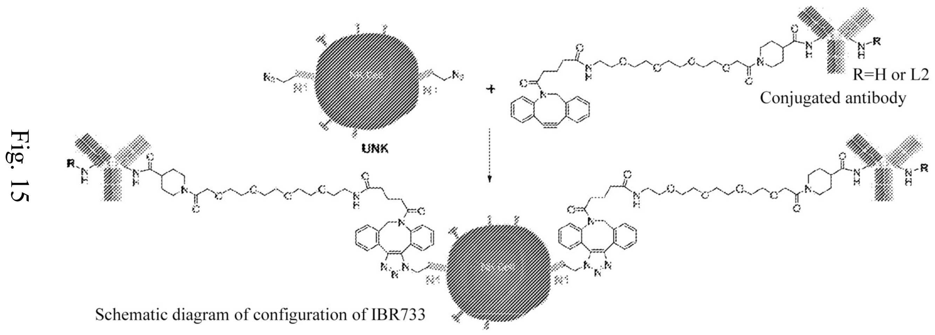

At last, in order to obtain the antibody-NK cell conjugate, the UNK and the L2-conjugated antibody can be subjected to a coupling reaction in the culture medium (the reaction mechanism is shown in FIG. 15) to obtain the antibody-NK cell conjugate.

Further, subsequent processes include the preparation of an antibody-NK cell conjugate formulation to effectively prolong the stability of the antibody-NK cell conjugate. The formulation may contain isotonic-maintaining components such as sodium chloride and human serum albumin, and may also contain components such as trehalose, sucrose, dextran, DMSO, and the like, which maintain the cells' tolerance to low temperature and the activity of proteins and enzymes.

In a second aspect, the present application provides a cell population comprising the antibody-natural killer cell (NK cell) conjugate of the first aspect.

In some embodiments, the count of CD3⁻CD56⁺CD16⁺ cells is at least 95% with respect to the total cell count in the cell population. In some embodiments, the count of CD3⁻CD56⁺CD16⁺ cells is at least 98% with respect to the total cell count in the cell population. In some embodiments, the count of CD3⁻CD56⁺NKG2D⁺ cells is at least 95% with respect to the total cell count in the cell population. In some embodiments, the count of CD3⁻CD56⁺NKG2D⁺ cells is at least 98% with respect to the total cell count in the cell population.

In some embodiments, the count of CD3⁺CD56⁺ cells is no more than 5% with respect to the total cell count in the cell population.

In some embodiments, the count of CD3-CD19⁺ cells is no more than 2% with respect to the total cell count in the cell population.

In some embodiments, the counts of CD3⁺CD4⁺ and CD3⁺CD8⁺ cells are no more than 2% with respect to the total cell count in the cell population.

In some embodiments, by cell count, the antibody-natural killer cell (NK cell) conjugate is at least 90% with respect to the total cell count in the cell population. In some embodiments, by cell count, the antibody-natural killer cell (NK cell) conjugate is at least 95% with respect to the total cell count in the cell population. In some embodiments, by cell count, the antibody-natural killer cell (NK cell) conjugate is at least 98% with respect to the total cell count in the cell population. In some embodiments, by cell count, the antibody-natural killer cell (NK cell) conjugate is at least 99% with respect to the total cell count in the cell population.

In a third aspect, the present application provides a pharmaceutical composition comprising the antibody-natural killer cell (NK cell) conjugate of the first aspect or the cell population of the second aspect, and a pharmaceutically acceptable carrier.

In some embodiments, the pharmaceutical composition comprises sodium chloride and/or human serum albumin.

In some embodiments, the pharmaceutical composition comprises trehalose, sucrose, dextran, DMSO, or any combination thereof.

In some embodiments, the pharmaceutical composition is used to treat acute myeloid leukemia (AML) in an individual.

In some embodiments, the pharmaceutical composition is used to treat an initially diagnosed acute myeloid leukemia.

In some embodiments, the pharmaceutical composition is used to treat relapse or refractory acute myeloid leukemia in an individual.

In some embodiments, the individual suffering from AML has received a chemotherapy and/or a targeted drug therapy. In some embodiments, the targeted drug is gilteritinib and/or venentoclax.

In some embodiments, the pharmaceutical composition is used in combination with venentoclax and/or azacitidine for the treatment of relapsed or refractory acute myeloid leukemia.

In a fourth aspect, the present application provides the use of the antibody-natural killer cell (NK cell) conjugate of the first aspect or the cell population of the second aspect in the manufacture of a medicament for the treatment of acute myeloid leukemia (AML) in an individual.

In some embodiments, the medicament is used to treat an initial diagnosed acute myeloid leukemia in an individual.

In some embodiments, the medicament is used to treat relapse or refractory acute myeloid leukemia in an individual.

In some embodiments, the individual has received a chemotherapy and/or a targeted drug therapy.

In some embodiments, the targeted drug is gilteritinib and/or venentoclax.

In some embodiments, the medicament is used in combination with venentoclax and/or azacitidine for the treatment of relapsed or refractory acute myeloid leukemia.

In a fifth aspect, the present application provides a method of treating acute myeloid leukemia (AML) in an individual, the method comprising administering to the individual an effective amount of the antibody-natural killer cell (NK cell) conjugate of the first aspect or the cell population of the second aspect or the pharmaceutical composition of the third aspect.

In some embodiments, the individual has an initially diagnosed acute myeloid leukemia.

In some embodiments, the individual has relapsed or refractory acute myeloid leukemia.

In some embodiments, the individual has received a chemotherapy and/or a targeted drug therapy.

In some embodiments, the targeted drug is gilteritinib and/or venentoclax.

In some embodiments, the method further comprises administering to the individual venentoclax and/or azacitidine for combination treatment of relapsed or refractory acute myeloid leukemia.

It is to be understood that the foregoing detailed description is intended only to enable those skilled in the art to have a clearer understanding of the present application and is not intended to be limiting in any way. Various modifications and variations can be made to the embodiments by those skilled in the art.

### Examples

The present application is further illustrated in connection with specific examples. It is to be understood that these examples are merely used to illustrate the present application and are not intended to limit the scope of the present application.

### Example 1 - Preparation of anti-CD33/CLL1 bispecific antibody-NK cell conjugates

The preparation of the antibody-NK cell conjugate in this example can generally be divided into the following steps:
(1) preparation of the anti-CD33/CLL1 bispecific antibody;
(2) preparation of the NK cell;
(3) preparation of the antibody linker (referred to as L2 linker in this example);
(4) preparation of the NK cell linker (referred to as N1 linker in this example);
(5) applying the antibody linker (L2 linker) to the antibody;
(6) applying the NK cell linker (N1 linker) to the NK cell; and
(7) conjugating the antibody to the NK cell, each carrying their respective linkers.

### (1) Preparation of anti-CD33/CLL1 bispecific antibody

Briefly, the methodology used to prepare the anti-CD33/CLL1 bispecific antibody in this example was to first prepare anti-CD33 and anti-CLL1 monoclonal antibodies, and then reduce the disulfide bond in the hinge region such that the arms of the monoclonal antibodies were separated and reassembled into the bispecific antibody (see FIG.4 for a schematic diagram of the reaction).

Specifically, the sequences of the anti-CD33 and anti-CLL1 monoclonal antibodies were obtained from phage library screening. The anti-CD33/CLL1 antibody was obtained by screening for the binding activity between the target proteins and cells expressing the target proteins, followed by confirmation through affinity studies. The anti-CLL1 monoclonal antibody included the heavy chain variable region set forth in SEQ ID NO:1 (HCDR1-3 being SEQ ID NO:15, 16, 17, respectively), the heavy chain constant region set forth in SEQ ID NO:2, the light chain variable region set forth in SEQ ID NO:3 (LCDR1-3 being SEQ ID NO:18, 19, 20, respectively), and the light chain constant region set forth in SEQ ID NO:4. The anti-CD33 monoclonal antibody included the heavy chain variable region set forth in SEQ ID NO:5 (HCDR1-3 being SEQ ID NO:9, 10, 11, respectively), the heavy chain constant region set forth in SEQ ID NO:6, the light chain variable region set forth in SEQ ID NO:7 (LCDR1-3 being SEQ ID NO:12, 13, 14, respectively), and the light chain constant region set forth in SEQ ID NO:8. The DNA sequences of the anti-CD33 and anti-CLL1 antibodies were determined, and recombinant plasmids expressing either the anti-CD33 monoclonal antibody or the anti-CLL1 monoclonal antibody, designated as IB12-CD33 and IB12-CLL1 in this example, were constructed.

The construction of monoclonal cell strains was based on known techniques, where the IB12-CD33 and IB12-CLL1 plasmids were electrotransfected into CHO cells, subjected to one round of minipool screening, followed by two rounds of screening after monoclones were plated, and confirmed by primary culture for passage stability, resulting in a cell strain stably expressing the anti-CLL1 monoclonal antibody and a cell strain stably expressing the anti-CD33 monoclonal antibody.

In the preparation of the anti-CD33-CLL1 bispecific antibody, the above-mentioned cell strains stably expressing the anti-CLL1 monoclonal antibody and the anti-CD33 monoclonal antibody were expanded, subjected to a mass culture, and collected for the unprocessed bulk (UPB), which underwent clarification and filtration to generate cell culture supernatants. The supernatants contained the anti-CD33 monoclonal antibody and the anti-CLL1 monoclonal antibody, respectively. Then, the anti-CD33 monoclonal antibody and the anti-CLL1 monoclonal antibody were subjected to a protein purification process via Protein A affinity purification, then assembled into the anti-CD33-CLL1 bispecific antibody under the conditions of reducing agent 2-MEA and air oxidation, and then subjected to a two-step chromatography of anion exchange and cation exchange to obtain the anti-CD33-CLL1 bispecific antibody.

### (2) Preparation of NK cell

PBMCs were derived from apheresis of peripheral blood lymphocytes from an allogeneic healthy donor. After separating from T cells and red blood cells, the cells were transferred to a primary cell cryopreservation solution and aliquoted according to the specification of ≥6.0×10⁷ viable cells per vial to obtain PBMCs. PBMCs were stored at ≤-175°C in liquid nitrogen for long-term storage.

PBMCs frozen in liquid nitrogen were removed, resuscitated in a suitable medium, cultured in an enlarged culture scale (i.e., expanded in an increased volume of culture medium) and added with NK cell-related cytokines which included but were not limited to IL2 and IL15, to maintain their proliferation capacity and activity. Exemplary NK cell culture stages, process parameters, and process control indicators are shown in Table 1 below.

**Table 1**

| Culture stage | Process parameters | Process control |
|---|---|---|
| Days 0-5 | 1. Temperature of resuscitation: 37°C | 1.PBMC resuscitation |
| Primary culture of NK cells | | Seeding density of viable cells: (1.00~2.00) ×10⁶ cells/mL |
| | Serum: 10% | |
| | Culture system: 120±15 mL Coating: incubation with coating factor combination at room temperature for at least 1 h or 2-8°C coating overnight. | |
| | | Cell viability: ≥80.0% |
| | | 2. Day 5: |
| | | Cell viability ≥60.0% |
| | 2. Culture conditions: | |
| | Temperature of 37±0.5°C, CO₂ concentration of 5±0.5% | |
| | 3. Fluid supplementation | |
| | Day 3: 54 mL NK cell activation medium and 6 mL serum (serum volume was 10% of the total volume of fluid supplementation) were added. | |
| Days 5-15 | 1. Fluid supplementation: | 1. Days 5, 7, and 9: |
| Expansion culture of NK cells | Day 5: After the cell suspension was homogeneously placed in a culture bag, NK cell activation medium and serum were added, with a total volume of the fluid supplementation of 100-420 mL (the volume of serum accounted for 10% of the total volume of the fluid supplementation). | density after the supplementation: ≥3.00×10⁵ cells/mL |
| | | 2. Day 9: |
| | | Cell viability ≥70.0% |
| | | Value of NK purity (CD3⁻ CD56⁺) was reported |
| | | Value of T-cell proportion (CD3⁺CD56⁻) was reported |
| | | 3. Days 13-15: |
| | Day 7: 10-30 mL serum was added, and NK cell expansion medium (containing 0.5% human albumin) was supplemented, with a total volume of the fluid supplementation of 100-700 mL. | Density after the supplementation: ≥6.00×10⁵ cells/mL |
| | | Cell viability: ≥ 75.0% |
| | | NK purity (CD3⁻CD56⁺) ≥85.0% |
| | | NKT cell proportion (CD3⁺CD56⁺) ≤4.0% Sterility test |
| | Day 9: Cell suspension was added at 1:3 into three cell culture bags, each supplemented with 200-800 mL NK cell expansion medium (containing 0.5% human albumin). | |
| | Day 11: 100-800 mL NK cell expansion medium (containing 0.5% human albumin) | |
| | Day 13-15: Each bag of cell suspension was split 1:2 into two cell culture bags, followed by addition of NK cell expansion medium. (containing 0.5% human albumin) | |
| | 2. Culture conditions: temperature of 37±0.5°C, CO₂ concentration of 5±0.5% | |

The quality control for the purity of final NK cells fulfilled the requirements specified in Table 2 below (other parameters, such as cell density and biosafety, complied with industry standards):

**Table 2.**

| Cell type | | Detection method | Standard |
|---|---|---|---|
| NK cell | NK: CD3⁻CD56⁺ | Flow cytometry | ≥95.0% |
| | NKT: CD3⁺CD56⁺ | | ≤5.0% |
| | CD3⁻CD56⁺CD16⁺ | | ≥95.0% |
| | CD3⁻CD56⁺NKG2D⁺ | | ≥95.0% |
| B cell (CD3⁻CD19⁺) | | | ≤2.0% |
| T cell (CD3⁺CD4⁺&CD3⁺CD8⁺) | | | ≤2.0% |

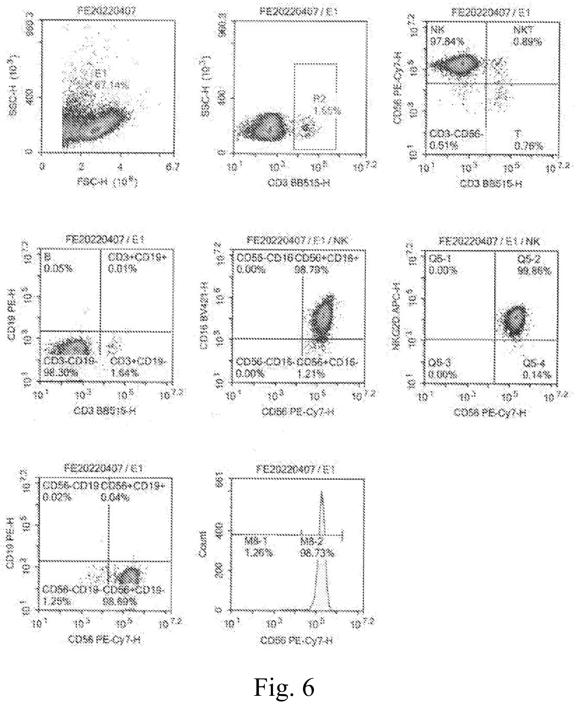

Figures 5 and 6 show results of flow cytometric detection of the immunological markers described above in the final prepared stock solution and the formulation of antibody-NK cell conjugates.

### (3) Preparation of antibody linker (referred to as L2 linker in this example)

The process for synthesizing L2 linker is shown in Figure 8, and the structural formula of L2 linker is shown in Figure 9. The process included three main synthetic steps: amide condensation, hydrolysis reaction, and synthesis of active ester.

### Step 1 - Amide condensation

Dibenzoazacyclooctyne glutaric acid (L2-1) and amino tetraethylene glycol methyl acetylpiperidinate (L2-2) were commercially available. An intermediate dibenzoazacyclooctyne glutaryl amino tetraethylene glycol methyl acetylpiperidinate (L2-3) was obtained according to the chemical condensation reaction. L2-3 is a stable compound.

Specifically, compound L2-1 and compound L2-2 (1:1.1, with L2-2 in excess) were dissolved in a dichloromethane (DCM) solution. Hydroxybenzotriazole (HOBt) and 1-ethyl-(3-dimethylaminopropyl)carbodiimide (EDCI) were separately added, followed by the addition of triethanolamine (TEA). The mixture was stirred at room temperature for 4-12 hours, quenched with water, extracted twice with dichloromethane (DCM), and purified with a silica gel column (dichloromethane: methanol = 20:1) to yield compound L2-3 as a yellow oil.

### Step 2 - Hydrolysis reaction

Intermediate L2-3 obtained in Step 1 was hydrolyzed to yield an intermediate dibenzoazacyclooctyne glutaryl amino tetraethylene glycol acetylpiperidinic acid (L2-4). The hydrolysis reaction was typically a quantitative reaction, and the product was used directly in the next synthesis step without further purification.

Specifically, compound L2-3 was dissolved in a mixed solution of methanol and water, cooled to 0°C, and then added with an aqueous solution of 1 mol/L lithium hydroxide (LiOH). The mixture was stirred for 4-12 hours with temperature increasing from 0°C to room temperature, acidified to pH between 2 and 3 with 1 mol/L HCl, extracted three times with ethyl acetate (EA) and dried to yield the intermediate dibenzoazacyclooctyne glutaryl amino tetraethylene glycol acetylpiperidinic acid (L2-4) as a yellow oil.

### Step 3 - Synthesis of active ester

Intermediate L2-4 obtained in Step 2 (without purification) was condensed with pentafluorophenol and dicyclohexylcarbodiimide (DCC) to yield L2, i.e., dibenzoazacyclooctyne-glutaryl-amino tetraethylene glycol-pentafluorophenyl acetylpiperidinate.

Specifically, compound L2-4 was dissolved in tetrahydrofuran (THF), cooled to 0°C, and then added with pentafluorophenol, hydroxybenzotriazole (HOBt), and dicyclohexylcarbodiimide (DCC). The mixture was stirred for 4-12 hours with temperature increasing from 0°C to room temperature, extracted three times with ethyl acetate (EA), dried and purified with a silica gel column (dichloromethane: methanol = 20:1) to yield dibenzoazacyclooctyne-glutaryl-amino tetraethylene glycol-pentafluorophenyl acetylpiperidinate (L2) as a yellow oil, the structural formula of which is shown in FIG. 9.

### (4) Preparation of NK cell linker (referred to as N1 linker in this example)

The process for synthesizing N1 linker is shown in FIG. 10, and the structural formula of N1 linker is shown in FIG. 11. The process included two main synthetic steps: amino azidoacetylation and hydroxyacetylation.

### Step 1 - Amino azidoacetylation

1.2 equivalents of a-azidoacetic acid (compound 1), 2 equivalents of hydroxybenzotriazole (HOBt), and triethylamine (Et₃N) were added to a solution of D-galactosamine hydrochloride (compound 2) in N,N-dimethylformamide (DMF). A small amount of methanol (MeOH) was added to assist dissolution. The reaction was performed at room temperature for 12 hours. The reaction mixture was poured into dichloromethane (DCM)/methanol (MeOH) and mixed by shaking. An ether was added to precipitate the oily product (compound 3), and the ether layer was decanted. This process was repeated twice, followed by vacuum drying. The product was used in the next reaction without further purification.

### Step 2 - Hydroxyacetylation

Compound 3 obtained above was added to anhydrous pyridine (Pyr.) and acetic anhydride (Ac₂O), followed by the addition of 4-dimethylaminopyridine (DMAP) catalyst at room temperature. The reaction was allowed to proceed for 12 hours. When the reaction was nearly complete, as detected by HPLC, the mixture was concentrated to obtain a racemic mixture of tetraacetyl-N-azidoacetyl-a,b-D-galactosamine. Ethyl acetate/petroleum ether was used to precipitate the product as a solid, mainly in a b-configuration, with a purity of >85%. Further purification was performed with a silica gel column (dichloromethane: methanol = 20:1), resulting in the N1 product with a single optical isomer purity greater than 90%. The two a,b- isomers were capable of being isomerized to each other in cells, converted to N-azidoacetyl sialic acid through multiple steps of metabolism and synthesis, and finally expressed on glycoproteins of the NK cell surface. The structural formula of N1 linker is shown in FIG. 11.

### (5) Applying antibody linker (L2 linker) to antibody

L2 linker was capable of generating a site-directed coupling reaction with the anti-CD33-CLL1 bispecific antibody in a system of PBS at pH 7.2-7.4, preferably HEPES at pH 7.2 (see FIG. 12 for a schematic illustration). Typically, one anti-CD33-CLL1 bispecific antibody was able to be conjugated to 1-4 (preferably 1-2) L2 linkers. The reaction was capable of being terminated by adjusting the pH to about 5.0 to yield a L2-conjugated antibody.

### (6) Applying NK cell linker (N1 linker) to NK cell

Once NK cells were cultured for 15-16 days, the culture medium was added with N1 linker and incubated for 12-18 hours to obtain N1 linker-modified NK cells (referred to as "UNK" in the examples of the present application) (see Figures 13 and 14 for a schematic diagram of the conjugation mechanism of N1 linker to NK cells and the structure of UNK).

### (7) Conjugating the antibody to the NK cell, each carrying their respective linkers

The UNK and the L2-conjugated antibody were subjected to a coupling reaction in the culture medium (see Figure 15 for the reaction mechanism) to obtain a stock solution (DS) of the antibody-NK cell conjugate. The antibody-NK cell conjugate was designated as "IBR733", which was used in the subsequent examples to refer to the anti-CD33-CLL1 bispecific antibody-NK cell conjugate.

Further, subsequent processes included the preparation of an antibody-NK cell conjugate formulation to effectively prolong the stability of the antibody-NK cell conjugate. The formulation could contain isotonic-maintaining components such as sodium chloride and human serum albumin, and could also contain components such as trehalose, sucrose, dextran, DMSO, and the like, which maintained the cells' tolerance to low temperature and the activity of proteins and enzymes.

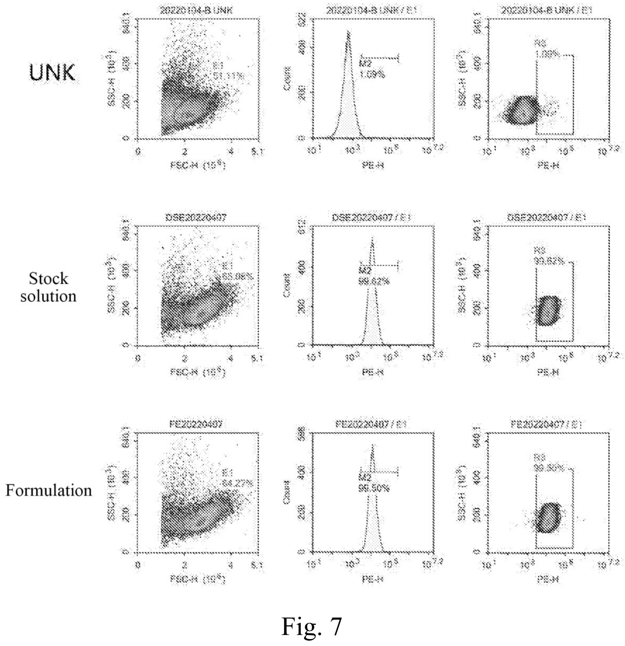

Purities of NK cells in the antibody-NK cell conjugate stock solution (FIG. 5) and the formulation (FIG. 6) were measured by flow cytometry. Results showed values of above 98%, which are larger than 95% as required by standards. In addition, the conjugation positivity rates of the antibody-NK cell conjugate stock solution and the formulation were assessed by flow cytometry, with UNK as a control. Results showed that the conjugation positivity rates of both the stock solution and the formulation were above 99% (FIG. 7), while the standard requirement was generally 90%.

### Example 2 - Comparison of primary antibody properties of conjugate IBR733 and anti-CD33-CLL1 bispecific antibody

In this example, conjugate IBR733 prepared in Example 1 was compared in terms of several primary antibody properties with the anti-CD33-CLL1 bispecific antibody prepared in Step (1) in Example 1. The results are shown in Table 3 below. Methodology for each test item followed conventional assays in the art.

**Table 3**

| Test item | | Anti-CD33-CLL1 bispecific antibody | Conjugate IBR733 |
|---|---|---|---|
| Isoelectric Point (by icIEF Method) | | N/A | Peak1: 8.430 |
| | | | Peak2: 8.556 |
| | | | Peak3: 8.661 |
| | | | Peak4: 8.775 |
| | | | Peak5: 8.862 |
| Stability (by DSF Method) | | Tₘ₁=72.2°C | Tₘ₁ = 67.2°C; |
| | | Tₘ₂=90.3°C | Tₘ₂ = 78.2°C |
| Affinity to target (by BLI method) | Human CD33 | 5.5×10⁻⁹ mol/L | 1.70×10⁻⁹ mol/L |
| | Human CLL1 | 2.6×10⁻⁸ mol/L | 7.63×10⁻⁹ mol/L |
| Affinity to Fc receptor (by BLI method) | FcγRI | N/A | 2.84×10⁻⁹ mol/L |
| | FcγRIIa | N/A | 3.44×10⁻⁷ mol/L |
| | FcγRIIIa (V176) | N/A | 1.50×10⁻⁷ mol/L |
| | FcγRIIIa (F176) | N/A | 9.68×10⁻⁸ mol/L |
| | FcRn | N/A | 2.41×10⁻⁸ mol/L |
| | C1q | N/A | 9.14×10⁻⁷ mol/L |

| | | | |
|---|---|---|---|
| Note: "N/A" means not tested. | | | |

As can be seen from the results in Table 3, conjugation of the anti-CD33-CLL1 bispecific antibody to the NK cell did not significantly affect the properties of the antibody itself, allowing its desired characteristics to be preserved.

### Example 3 - Evaluation of in vitro killing activity of conjugate IBR733

In the evaluation of cell-killing activities in this example, three tumor cell lines, U937, THP-1 and HL-60, which highly express CD33 and CLL1, were selected, and their levels of CD33 and CLL1 expression were pre-determined (by flow cytometry) and as shown in Table 4 below.

**Table 4**

| Tumor cell line | CD33 expression rate | CLL1 expression rate |
|---|---|---|
| U937 | 99.31% | 97.63% |
| THP-1 | 95.40% | 99.10% |
| HL 60 | 99.95% | 97.61 % |

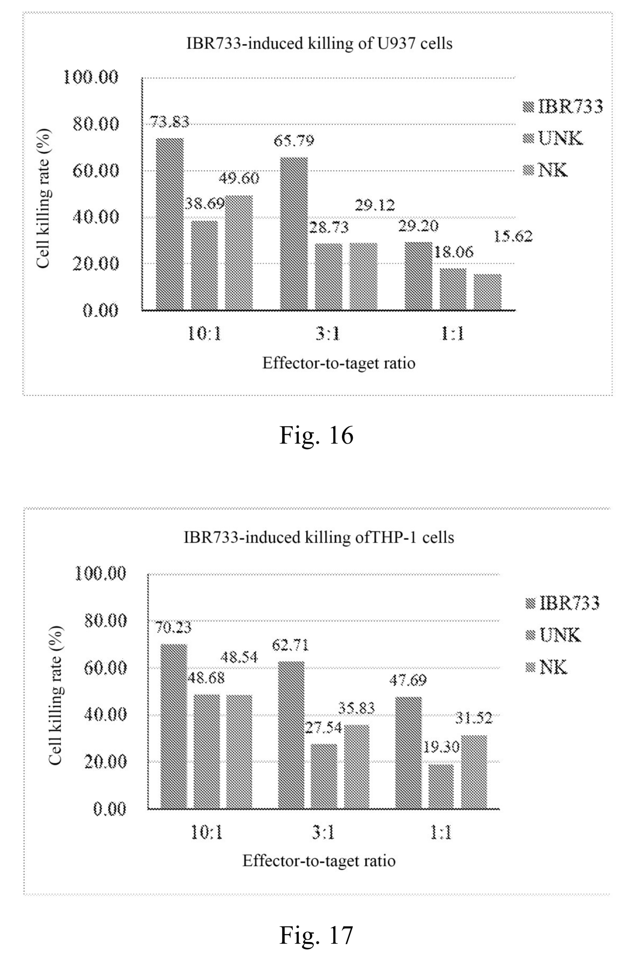

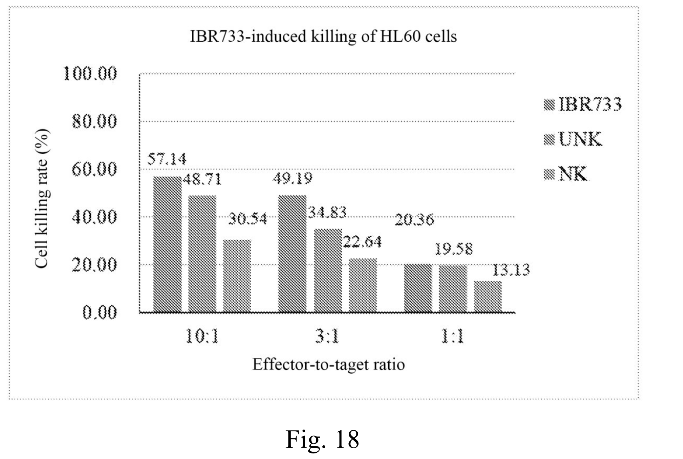

First, the cell-killing activities of conjugate IBR733, and the NK cells (obtained in step (2) of Example 1) and the UNK cells (obtained in step (6) of Example 1) as controls, against tumor cell lines U937, THP-1, and HL60, were measured by a Calcein AM release assay. A maximum release control, a spontaneous release control, and a medium control were included in the experiment. Each of the NK cells, the UNK cells, and Conjugate IBR 733 were set at three concentrations, with three replicate wells per concentration. The tumor cells were stained with Calcein AM, and then co-incubated with the NK cells, the UNK cells, and IBR733 for 4 hours, followed by a centrifugation to collect supernatants. Changes in fluorescence intensity were detected with a microplate reader, and percentages of cell killing were calculated. Results are shown in FIG. 16 to FIG. 18. The results indicate that the killing activity of IBR733 against U937, THP-1, and HL60 cells was significantly higher than that of the NK cells and UNK cells.

Next, the dose-response relationship of IBR733 induced killing of U937, THP-1, and HL60 cells was measured with the Calcein AM release method. Nine concentrations were set for IBR733, with six replicate wells per concentration. Changes in fluorescence intensity were detected with a microplate reader, and cell-killing rates and EC₅₀ values were calculated. Multiple batches of IBR733 were prepared at different times and tested for killing effects on tumor cells U937, THP-1, and HL-60. Results are shown in Table 5 below.

The results indicated that the multiple batches of IBR733 had significant killing effects on all three tumor cell lines, and the effects were relatively stable. IBR733 induced killing of U937 cells with EC₅₀ values between 0.6~0.8, and maximum inhibition rates of above 90%. IBR733 induced killing of THP-1 cells with EC₅₀ values between 0.8~1.6, and maximum inhibition rates of above 75%. IBR733 induced killing of HL 60 cells with EC₅₀ values between 0.7~4.0, and maximum inhibition rates of above 80%.

**Table 5**

| **Tumor cell line** | **Tumor cell generation** | **IBR733 Batch Date** | **EC₅₀ (effector-to-target ratio) average** | **Maximum inhibition rate (%)** |
|---|---|---|---|---|
| U937 | F14 | June 8, 2021 | 0.71 | 94.42 |
| | F14 | June 9, 2021 | 0.66 | 91.37 |
| | F15 | June 10, 2021 | 0.69 | 102.03 |
| THP-1 | F7 | July 2, 2021 | 0.84 | 95.50 |
| | F7 | July 2, 2021 | 1.09 | 96.21 |
| | F7 | July 6, 2021 | 1.52 | 75.69 |
| HL 60 | F4 | July 7, 2021 | 0.76 | 107.06 |
| | F8 | July 12, 2021 | 3.89 | 82.36 |

### Example 4 - Evaluation of factor release function of conjugate IBR733

In this example, a functional study of cytokine secretion caused by conjugate IBR733 was carried out. The investigated cytokines included IL-2 (interleukin-2), IL-6 (interleukin-6), IL-15 (interleukin-15), IL-1β (interleukin-1β), TNFα (tumor necrosis factor α), IFNγ (interferon-γ); IL-8 (interleukin-8), CCL2 (chemokine 2), CCL3 (chemokine 3) and CCL5 (chemokine 5). The measurement of the cytokines was performed by using commercial ELISA reagent kits and testing supernatants of various cell cultures according to the manufacturer's instructions.
(1) After conjugate IBR733 was co-incubated with two tumor effector cells (THP-1, U937) at different effector-to-target ratios (1: 1, 3: 1, 10: 1, and 30:1), secretion levels of IL-2, IL-6, IL-15, and IL-1β in cell culture supernatants were determined by ELISA. Results are shown in Table 6 and Table 7. The results indicate that the secretion levels of IL-2, IL-6, IL-15, and IL-1β were all lower than the average levels in healthy individuals. IL-2 factor levels were all less than 120 pg/ml and in a dose-dependent manner, and were lower than the IL-2 level of 9.40±2.31 ng/ml in healthy human serum. IL-6 factor levels were all less than 2.5 pg/ml, and lower than the IL-6 level of 60.32±3.24 pg/ml in healthy human serum. IL-15 factor levels were all less than 0.2 pg/ml, and lower than the IL-15 level of 13.38±4.41 pg/ml in healthy human serum. The levels of IL-1β secretion were all less than 0.5 pg/ml.

**Table 6**

| Effector-to-target ratio (IBR733: THP-1 cells) | Factor level (pg/ml) | | | |
|---|---|---|---|---|
| | IL-2 | IL-6 | IL-15 | IL-1 β |
| 30: 1 | 104 | 0.478 | 0.047 | 0.248 |
| 10: 1 | 44.2 | 0 | 0.114 | 0.172 |
| 3: 1 | 18.8 | 0.101 | 0.065 | 0.380 |
| 1: 1 | 8.27 | 0.323 | 0.118 | 0.292 |

**Table 7**

| Effector-to-target ratio (IBR733:U937 cells) | Factor level (pg/ml) | | |
|---|---|---|---|
| | IL-2 | IL-6 | IL-15 |
| 30: 1 | 87 | 2.18 | 0.033 |
| 10: 1 | 50.9 | 0.885 | 0 |
| 3: 1 | 16 | 0.36 | 0.066 |
| 1: 1 | 7.33 | 0.198 | 0 |

(2) Conjugate IBR733 was co-incubated with U937 and THP-1 tumor cells at effector-to-target ratios of 30:1, 10: 1, 3: 1, 1: 1. Secretion levels of TNFα and IFNγ were determined by ELISA. As shown by the results in Table 8, after the co-incubation of IBR733 with the tumor cells, the secretion level of TNFα did not change significantly by an order of magnitude, and was lower than the average level of 22.75±6.28 µg/ml in healthy people. The secretion level of IFNγ was positively correlated with the doses of effector-to-target ratios. The secretion level of IFNγ was significantly up-regulated after the co-incubation of IBR733 with tumor cells, indicating a certain correlation with the killing effects on the tumors.

**Table 8**

| Effector-to-target ratio (IBR733: Tumor cells) | TNFα factor level (pg/ml) | | INFγ factor level (pg/ml) |
|---|---|---|---|
| | U937 | THP-1 | U937 |
| 30: 1 | 225 | 214 | / |
| 10: 1 | 92.8 | 112 | 353 |
| 3: 1 | 44.6 | 35.5 | 127 |
| 1: 1 | 20.0 | 18.4 | 50.7 |

(3) Secretion levels of CCL2, CCL3, CCL5, and IL-8 (CXCL8) in the cell culture supernatants were determined by ELISA after the co-incubation of conjugate IBR733 with U937 and THP-1 tumor cells at different effector-to-target ratios (1:1, 3:1, 10:1, and 30:1). As shown by the results in Table 8 and Table 9, the levels of CCL3, CCL5, and IL-8 factors were positively correlated with exposure doses of IBR733. CCL3, CCL5, and IL-8 were possible contributions to the tumor cell-killing effect of the IBR733 conjugate, which were achieved by mediating chemotaxis of related immune cells such as macrophages and leukocytes. The secretion levels of CCL2 were negatively correlated with the exposure doses of IBR733, possibly because the tumor cells were able to secrete CCL2 to achieve migration and invasion, while NK cells inhibited the chemotactic capability of the tumor cells, as evidenced by the decrease in CCL2 concentrations with increasing effector-to-target ratios of NK cells.

**Table 9**

| Effector-to-target ratio (IBR733: U937 cells) | Factor level (pg/ml) | | |
|---|---|---|---|
| | CCL2 | CCL3 | CCL5 |
| 30: 1 | 95.6 | 10170 | 4975 |
| 10: 1 | 561 | 7388 | 3798 |
| 3: 1 | 1707 | 2038 | 1363 |
| 1: 1 | 2106 | 773 | 518 |

**Table 10**

| Effector-to-target ratio (IBR733: THP-1 cells) | Factor level (pg/ml) | | | |
|---|---|---|---|---|
| | IL-8 | CCL2 | CCL3 | CCL5 |
| 30: 1 | 491 | 23.6 | 11066 | 5967 |
| 10: 1 | 187 | 35.2 | 7806 | 3297 |
| 3: 1 | 77.5 | 89.3 | 2945 | 1240 |
| 1: 1 | 52.8 | 258 | 1245 | 455 |

(4) Conjugate IBR733 was co-incubated with U937 and THP-1 tumor cells at different effector-to-target ratios of 10:1, 3:1, and 1:1 (while a control was set up to contain conjugate IBR733 at the same effector-to-target ratios but without tumor cells). Cell culture supernatants were collected and measured for secretion levels of granzyme B and perforin by ELISA. As shown by the results in Table 11 and Table 12, IBR733 alone secreted a certain amount of granzyme B and perforin in a dose-dependent manner. When IBR733 was co-incubated with U937 and THP-1 cells, granzyme B expression levels were upregulated after tumor stimulation, while the secretion levels of perforin did not change significantly.

**Table 11**

| Effector-to-target ratio | Granzyme B level (pg/ml) | |
|---|---|---|
| (IBR733: Tumor cells) | U937 cells | THP-1 cells |
| IBR733-10: 0 | 1961.863 | |
| IBR733-3: 0 | 445.725 | |
| IBR733-1: 0 | 174.629 | |
| IBR733: Tumor Cells -10: 1 | 2153.249 | 2229.305 |
| IBR733: Tumor Cells -3: 1 | 454.072 | 460.044 |
| IBR733: Tumor Cells -1: 1 | 190.515 | 196.67 |

**Table 12**

| Effector-to-target ratio (IBR733: Tumor cells) | Perforin level (pg/ml) | |
|---|---|---|
| | U937 cells | THP-1 cells |
| IBR733-10 | 3700.954 | |
| IBR733-3 | 1549.247 | |
| IBR733-1 | 703.097 | |
| IBR733: Tumor Cells -10: 1 | 4366.723 | 4168.727 |
| IBR733: Tumor Cells -3: 1 | 1722.815 | 1857.575 |
| IBR733: Tumor Cells -1: 1 | 808.464 | 832.199 |

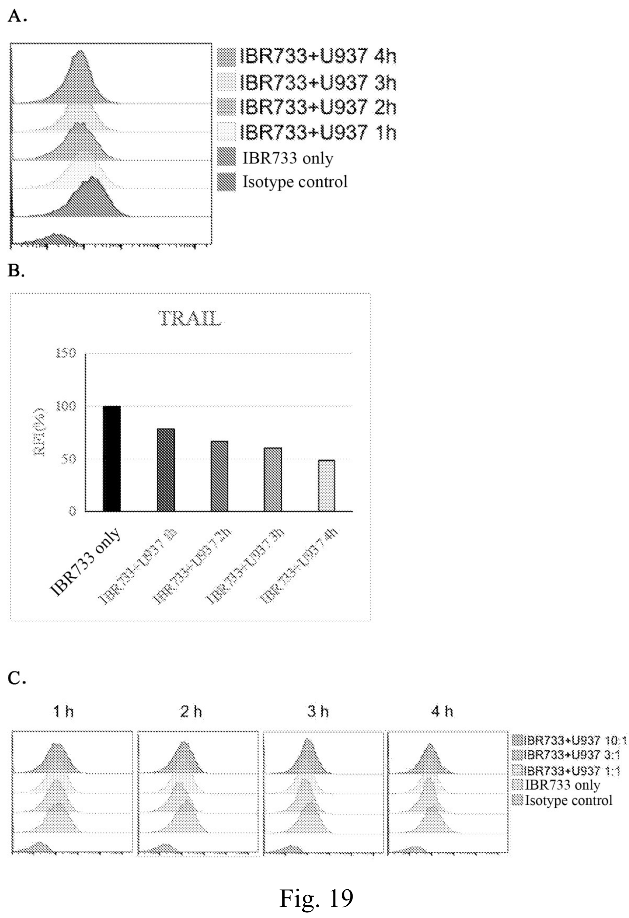

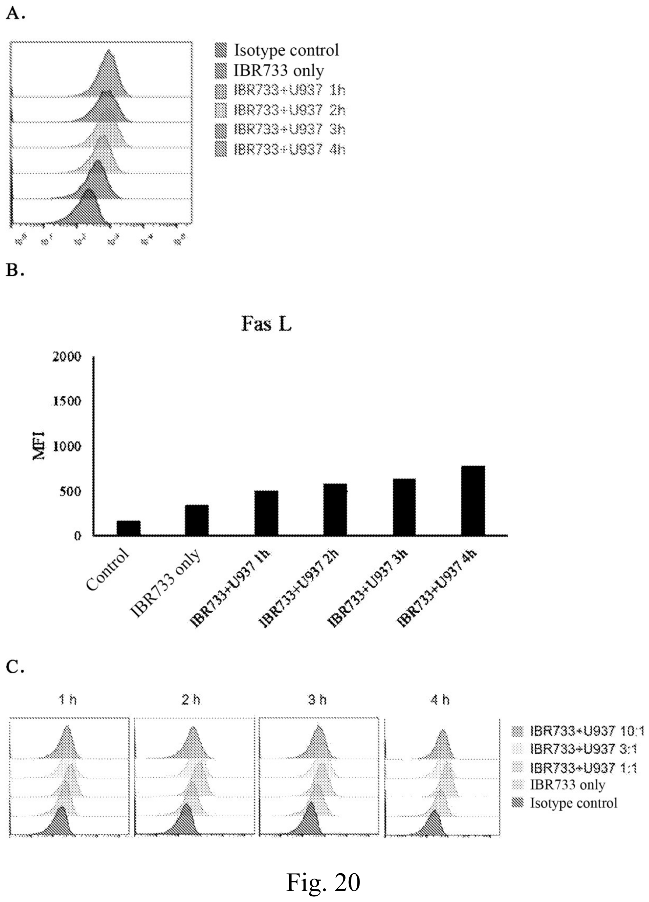

(5) Secretion levels of FasL/TRAIL factor after the co-incubation of IBR733 with U937 tumor cells were measured by flow cytometry. IBR733 and U937 tumor cells were co-incubated for 1 h, 2 h, 3 h, and 4 h, respectively. Changes in expression levels of TRAIL and FasL on the surface of IBR733 cells were analyzed by flow cytometry. When U937 tumor cells were killed by IBR733, the expression of FasL and TRAIL on the surface of NK cells changed over time, increasing first and then decreasing, without a significant correlation with doses. Results are shown in FIG. 19 and FIG. 20.

### Example 5 - Efficacy study of conjugate IBR733 on U937 tumor-bearing mouse model

### Pharmacodynamics PD

In the establishment of a model for *in vivo* pharmacodynamic assay, the U973 tumor strain, which was well-inhibited by IBR733 *in vitro,* was inoculated into NPG mice with a loss of NK cell functions caused by gene knockout. Considering the absence of cytokines such as IL-15 in the test animals, NPG mice, in order to mimic the maintenance of NK cells *in vivo,* IL-15 was added exogenously in the *in vivo* pharmacodynamic assay to prolong the persistence of IBR733 in the animals. Therapeutic effects of IBR733 on U937 cell (human histiocytic lymphoma cells) NPG tumor-bearing mice were observed. Dose-effect relationship was explored, and the primary *in vivo* pharmacodynamic effects of IBR733 against tumors were evaluated.

Ninety-one NPG mice were inoculated with U937 cells via tail vein, and were randomly divided into seven groups according to their body weights two days after inoculation. Groups 1 to 7 were a vehicle control group, an anti-CD33-CLL 1 bispecific antibody control group (7.5 mg/kg), an IL-15 group (0.5 µg/mouse), an IL15 + IBR733 low dose group (2.5×10⁸ cells/kg), an IL15 + IBR733 medium dose group (5.0×10⁸ cells/kg), an IL15 + IBR733 high dose group (1.0×10⁹ cells/kg), and an IBR733 medium dose group (5.0×10⁸ cells/kg), respectively, with 13 mice per group. IBR733 (administrated via tail vein injection) was administered on days 1, 2, 8, and 9 after tumor inoculation. For the treatment group involving IL-15, IL-15 (0.5 µg/mice) was administered intraperitoneally daily from day 1 to day 13 after tumor inoculation. Weighing was performed once before the grouping, and twice a week after administration. Bioluminescence signals were detected. Blood was collected and measured for the proportion of lymphocyte subsets. The last three animals of each group were not administrated, and their blood was collected and detected for the proportion of CD33/CLL1 cells two days after inoculation.

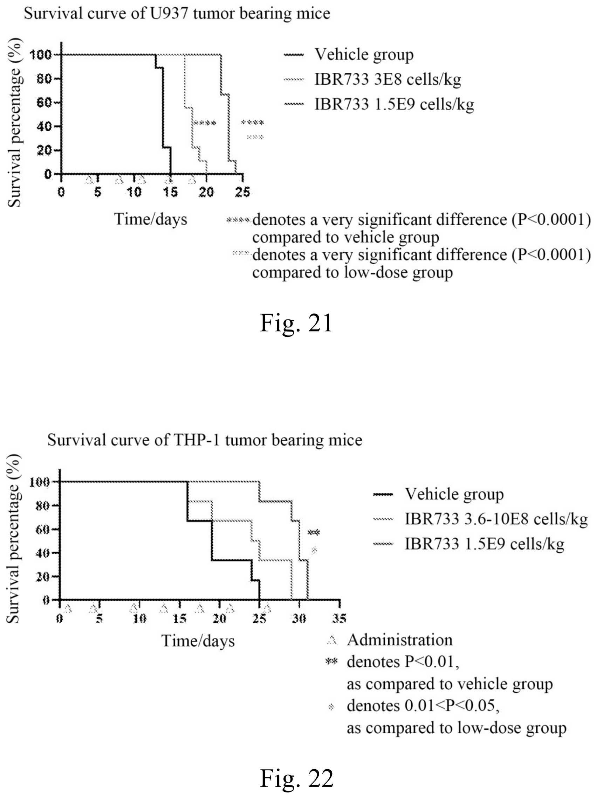

The results of the study showed the following.
1) The proportions of CD33/CLL1 cells in the blood collected from the last three animals of each group were basically the same, and there was no difference in the proportions of tumor cells in each group. The results suggested that the NPG mouse model with venously transplanted tumor of U937 cells (human histiocytic lymphoma cells) of the *in vivo* pharmacodynamic assay had been successfully established, and the proportions of tumor cells were basically the same at the time of grouping.
2) The IL15 + IBR733 high dose group showed significantly prolonged survival of the test animals compared with the vehicle control group and other groups, and showed dose dependence. The bioluminescence intensity of the tumor cells as measured beginning on day 7 showed that the mean bioluminescence intensity of the tumor cells of the IL-15 + IBR733 high dose group was reduced by about 13.6 times compared with the vehicle control group, with a significant difference (p < 0.001), showing an inhibitory effect on tumor growth.
3) According to observations of death/moribund, animals in the IL15 + high dose group showed prolonged survival (p<0.001). The longest survival time of the tested animals was 21 days. Animal deaths began on day 10 and all died by day 13 in the vehicle control group. Animal deaths began on day 12 and all died by day 14 in the antibody control group. Animal deaths began on day 10 and all died by day 12 in the IL-15 group. Animal deaths began on day 10 and all died by day 13 in the IL-15 + IBR733 low dose group. Animal deaths began on day 12 and all died by day 13 in the IL-15 + IBR733 medium dose group. Animals deaths began on day 11 and all died by day 13 in the IBR733 medium dose group. Animal deaths began on day 12 and all died by day 21 in the IL-15 + IBR733 high dose group. The results are shown in Figure 21.

The *in vivo* pharmacodynamic assay showed that U937 cells were able to proliferate in NPG mice after intravenous inoculation. Intravenous administration of IBR733 at a high dose of 1.0×10⁹ cells/kg in combination with IL-15 twice a week for two consecutive weeks was able to significantly inhibit the proliferation of U937 cells and significantly prolong the survival of the animals. The animals in this group were generally in better conditions, and had no significant difference in body weight from those in the other dose groups and the control group, with only a slight loss of weight, indicating good efficacy, safety and tolerance, as well as certain reference values for further clinical applications.

### Pharmacokinetics PK

IBR733 may exist in two forms after being injected into animals: IBR733 [CD3⁻ CD56⁺(CD33-CLL1)⁺] cells, i.e., unmetabolized IBR733 cells, and NK [CD3⁻ CD56⁺(CD33-CLL1)⁻] cells where the conjugated antibodies have detached, i.e., IBR733 metabolized cells. Positivity ratios of IBR733 (CD33-CLL1-raNK) and total NK cells were detected by flow cytometry to evaluate the distribution and survival time of the cells in animals.

A total of 56 NPG mice as a model with venously transplanted tumors of human U937-luc (a half being male, and the other half being female) were randomly divided into G1 group (vehicle control) of 8 mice and G2 group (IBR733, 5.0×10⁷/mouse, i.v., administrated once + IL-15, 1.8 µg/mouse, i.p., QW) of 48 mice on day 5 according to body weight. Peripheral blood, lung, spleen, and bone marrow samples were collected at designated time points after grouped administration. The proportions of IBR733 cells and total NK cells in the blood were measured by flow cytometry (FACS). Blood was collected in batches from mice in G1 group at 1 h, 3 d, 7 d and 11 d, and from mice in G2 group at 1 h, 4 h, 8 h, 1 d, 3 d, 7 d and 11 d. A fresh tissue sample was collected from each of the lung, spleen and bone marrow of G1 group at 11 d and of G2 group at 1 h, 8 h, 1 d, 3 d, 7 d and 11 d after administration (8 mice at each time point, a half of which was male, and the other half of which was female).

In the mice model with venously transplanted tumors of human U937-luc, 1~8 h after the injection of IBR733 into the mice, IBR733 and NK total cells were detectable in peripheral blood, bone marrow, lung, and spleen. The amount of IBR733 distributed among tissues varied. Enrichment was more significant in the lung than the spleen. The survival time of IBR733 in each tissue was about 168 h. In peripheral blood, it peaked at 4 h after administration and then gradually decreased. In bone marrow, it continuously rose for 72 h to the peak after administration and then gradually decreased. In lung and spleen, it peaked at 8 h after administration and then gradually decreased. Enrichment was more significant in lung cells than spleen cells. No mice died unexpectedly during the experiment, showing good safety.

### Example 6 - Efficacy study of conjugate IBR733 on THP-1 tumor-bearing mouse model

In the establishment of a model for *in vivo* pharmacodynamic assay, human monocytic leukemia cells (THP-1) were inoculated in NCG mice with a loss of NK cell functions caused by gene knockout. Therapeutic effects of IBR733 on THP-1 cell tumor-bearing NCG mice were observed. Dose-effect relationship was explored. The primary *in vivo* pharmacodynamic effects of IBR733 against tumors were evaluated.

Screening and grouping: 48 female NCG mice D8 were each inoculated with 5.0×10⁶ THP-1 cells. Three NCG mice were randomly selected on D1, and were detected for tumor cells in peripheral blood and femoral bone marrow by flow cytometry. Once tumor cells were detected, the mice were grouped according to body weight on D2. They were divided into two experiments, P1 and P2, with 27 mice in experiment P1 for tumor cell detection, and 18 mice in experiment P2 for survival observation. Experiments P1 and P2 each included a vehicle control group, a low-dose group (3.6~10×10⁸ cells/kg), and a high-dose group (1.5×10⁹ cells/kg), with 9 mice/group in experiment P1, and 6 mice/group in experiment P2. The day of peripheral blood and tissue detection was D1.

Administration manner. The test substance was administered to the low and high dose groups of experiment P1 on D2, D5, D9, and D12. The test substance was administered to the low and high-dose groups of experiment P2 on D2, D5, D9, D12, D16, D19, D23, and D26. The test substance was administered via tail vein injection. The vehicle control groups in experiments P1 and P2 were respectively administered with the buffer for the formulation on the date of administration of the test substance, and the date of administration was D2.

Detection index. Blood and femoral bone marrow were collected on D1, D5, D9, and D12 of experiment P1, and were detected for THP-1 tumor cells by flow cytometry. For experiment P2, all the animals were observed twice a day during the experiment, with survival as the detection index. Survival curves were plotted at the end of the experiment.

The results of the study showed the following.
1) Results of flow cytometry. Tumor cells were detected in the peripheral blood and bone marrow of the animals in each group on D1 before administration, and there was no significant difference in the proportion of the tumor cells detected on D1 among the vehicle control group, the test substance low-dose group and the test substance high-dose group. Therefore, the modeling was considered to be successful. On D5 and D9 after administration, the tumor cell counts in peripheral blood showed a decreasing trend in all groups, while in femoral bone marrow, they showed an increasing trend. However, the tumor cell counts in both of the low-dose and high-dose groups were lower than those in the vehicle control group. Moreover, the tumor cell counts in bone marrow of both of the low-dose and high-dose groups were significantly (P<0.05) lower than those in the vehicle control group. The tumor cell inhibition was positively correlated with IBR733 cell dose.
2) General clinical observations. In experiment P2, by D31, all animals in the vehicle control group, the low-dose group, and the high-dose group had died. The median survival time for each group was 20, 25.5, and 31 days, respectively. Compared with the vehicle control group, the high-dose group showed a significant increase in survival time (P<0.01), with an increase rate of 55.00%. Compared with the low-dose group, the high-dose group showed a significant increase in survival time (P<0.05), with an increase rate of 21.57%. The results are shown in Figure 22.

Conclusions. The *in vivo* pharmacodynamic experiments showed that human monocytic leukemia cells (THP-1) were able to proliferate in NCG mice after intravenous inoculation. Intravenous administration of 3.6-10×10⁸ cells/kg and 1.5×10⁹ cells/kg of the test substance twice a week for four consecutive weeks can inhibit the proliferation of human monocytic leukemia cells (THP-1) in animals. The tumor cell inhibition rate was positively correlated with the dose of IBR733 cells, and intravenous administration of 1.5 ×10⁹ cells/kg of the test substance was able to significantly prolong the survival time of the mice.

### Example 7 - Clinical study of conjugate IBR733 in treatment of recurrent/refractory acute myeloid leukemia

In this example, the study population in the clinical trial suffered from an indication designated for IBR733, that is, it included subjects diagnosed with relapsed/refractory acute myeloid leukemia (AML) according to the "Chinese Guidelines for Diagnosis and Treatment of Relapsed/Refractory Acute Myeloid Leukemia (2017 Edition)". A total of 12 subjects were enrolled, including 9 males (75.0%) and 3 females (25.0%), with a median age of 52.5 years (ranging from 23-68 years), which includes, by AML FAB classifications, 7 cases of AML (M2), 2 cases of AML (M5), 2 cases of AML (M1) transformed from MDS, and 1 case of AML (M1). In addition to AML, the subjects had at least one or more concomitant diseases or medical histories. According to literature reports, the incidence of AML increases with age, showing a significant increase starting at age 50, peaking at ages 60-69, with a significantly higher incidence in males than females and being rare in children. Therefore, the study population in this trial was designed to cover the main patient population, and subjects enrolled so far and subsequently were representative at some degrees, enabling the test drug to exert its maximum effect and allowing for a more accurate evaluation of its efficacy. In the future, as needed, relevant clinical trials in underage populations (under 18 years old) may be conducted as appropriate.

With respective to the trial design, primary objectives were to observe safety and tolerability, while secondary objectives were to observe efficacy indicators such as overall survival (OS) and composite complete response rate (CR+CRi). The dose-escalation principle was adopted to preliminarily observe the correlation between the present cellular product and adverse events, and to observe expected efficacy based on this. While obtaining first-hand data on safety and tolerability, a basis for dose setting in later trials was also provided.

Twelve subjects enrolled in the trial receiving a treatment with an IBR733 injection had previously undergone a combined chemotherapy or a targeted drug therapy for 0.5 to 4 or more years, with their conditions not effectively controlled. Clinically, they had developed resistance to the chemotherapy or could not tolerate it. Nine subjects had previously received a chemotherapy combined with a venentoclax targeted therapy but failed, including one subject (01-004) who received a chemotherapy combined with two targeted drugs (gilteritinib + venentoclax).

As of the data cutoff date of March 1, 2022, 12 subjects received varying cycles of treatment with the IBR733 cell injection, ranging from 1 to 14 cycles. 2 subjects achieved PR, 2 subjects had stable disease (SD), 4 subjects had PD, and 4 subjects were not able to be evaluated. In terms of survival, the longest survival time was 14 months, and this subject continued to benefit. Specific efficacy assessment of this study was as follows.

Three subjects (01-001, 01-002, 01-008) completed 2 to 14 cycles of treatment with the IBR733 cell injection, showing varying degrees of improvement, with clinical benefits observed in different treatment cycles.

Subject 01-001 received 14 cycles of treatment, with an efficacy assessment of PR, and continued to benefit clinically, with a survival time of 14 months as of the data cutoff date of March 1, 2022.

Subject 01-002 received 5 cycles of treatment, with an efficacy assessment of PR, and subsequently received allogeneic bone marrow transplantation, with a survival time of 13 months as of the data cutoff date of March 1, 2022.

Subject 01-008 received 2 cycles of treatment, with the disease under control and in a stable state.

Two subjects (01-005, 01-006) showed increased bone marrow blast cells before the second cycle of treatment, and received a combined chemotherapy. However, both patients did not return to the hospital for follow-ups and further treatment according to the treatment cycle, were not able to be evaluated, and withdrew from the study on their own.

Three subjects (01-009, 01-011, 01-012) showed disease progression after 1 to 4 cycles of treatment with the IBR733 cell injection. Subject 01-009 planned to undergo allogeneic hematopoietic stem cell transplantation after 1 cycle of treatment. Subjects 01-011 and 01-012 received 3 and 4 cycles of treatment, respectively, and withdrew from the study due to disease progression.

Four subjects (01-003, 01-004, 01-007, and 01-010) were in the very late stage of the disease in clinic, and received 1 cycle of treatment. The treatment process went well. However, due to personal reasons, they did not return to the hospital for follow-up and treatment according to the treatment cycle, and withdrew from the study. Their conditions were not able to be evaluated.

Details of the treatment and results of the efficacy assessment of the 12 subjects are shown in Table 13 below.

**Table 13**

| **Basic information of subjects** | **Administra tion time** | **Cell count/cy cle** | **Combinat ion therapy** | **Blast cells after treatment (%)** | **Efficacy assessm ent** | **OS (Mont h)** |
|---|---|---|---|---|---|---|
| | C1 (2021.01.11-15) | 1.54×10⁹ | / | 26.5→28.0 | SD | |
| | C2 (2021.02.03-05) | 3.32×10⁹ | / | 20.5 | SD | |
| NO. 001, FEMALE, 68, AML (M2a) | C3 (2021.03.13-15) | 6.50×10⁹ | / | 22.0 | SD | 14* |
| | C4 (2021.04.25-28) | 6.26×10⁹ | / | 27.5 | SD | |
| | C5 (2021.05.27-29) | 5.97×10⁹ | / | 20.0 | SD | |
| | C6 (2021.07.02-04) | 6.30×10⁹ | / | 30.5 | SD | |
| | C7 | 6.30×10⁹ | / | 13.0 | PR | |
| | (2021.08.20-23) | | | | | |
| | C8 (2021.09.22-24) | 6.67×10⁹ | / | 41.5 | PD | |
| | C9 (2021.10.15-17) | 6.36×10⁹ | Venentoclax | 20.0 | PR | |
| | C10 (2021.11.10-13) | 6.51×10⁹ | Venentoclax | 18.0 | PR | |
| | C11 (2021.12.09-11) | 6.83×10⁹ | Venentoclax | 14.0 | PR | |
| | C12 (2021.12.30-01) | 8.30×10⁹ | Venentoclax | 24.0 | PR | |
| | C13 (2022.01.20-22) | 9.20×10⁹ | Venentoclax | 21.5 | PR | |
| | C14 (2022.02.22-24) | 8.80×10⁹ | Venentoclax | UD | NE | |
| | C1 (2021.02.05-09) | 1.72×10⁹ | / | 24.5→14.0 | SD | |
| | C2 (2021.03.13-15) | 6.50×10⁹ | / | 17.0 | SD | |
| NO. 002, MALE, 56, AML (M1) | C3 (2021.04.09-12) | 5.46×10⁹ | / | 6.0 | PR | 13* |
| | C4 (2021.05.08-10) | 5.58×10⁹ | / | 20.0 | PD | |
| | C5 (2021.06.09-11) | 5.79×10⁹ | / | bone marrow transplantat ion | NE | |
| NO. 003, FEMALE, 56, AML (M1, MDS TRANSFORM ED) | C1 (2021.05.28-30) | 5.27×10⁹ | / | UD | NE | 1 |
| NO. 004, FEMALE, 23, AML (M5) | C1 (2021.06.17-19) | 4.40×10⁹ | / | UD | NE | 1.5 |
| NO. 005, MALE, 56, AML (M2) | C1 (2021.06.25-27) | 5.58×10⁹ | / | 6.0-56.5 | PD | 4 |
| | C2 (2021.07.27-29) | 5.80×10⁹ | combined chemothera py | UD | NE | |
| NO. 006, MALE, 44, AML (M2) | C1 (2021.07.03-05) | 5.02×10⁹ | / | 36-76 | PD | 2.5 |
| | C2 (2021.08.11-13) | 5.60×10⁹ | combined chemothera py | UD | NE | |
| NO. 007, MALE, 65, AML (M2) | C1 (2021.07.05-07) | 4.90×10⁹ | / | UD | NE | 5.5 |
| NO. 008, MALE, 42, AML (M2) | C1 (2021.07.08-10) | 5.06×10⁹ | / | 62→40.5 | SD | 2.5 |
| | C2 (2021.08.18-20) | 5.40×10⁹ | combined chemothera py | UD | NE | |
| NO. 009, MALE, 46, AML (M5) | C1 (2021.07.13-15) | 5.19×10⁹ | / | 49→91.6 bone marrow transplantat ion | PD | 8* |
| NO. 010, MALE, 47, AML (M1, MDS TRANSFORME D) | C1 (2021.07.14-16) | 5.32×10⁹ | / | UD | NE | 6.5 |
| NO. 011, MALE, 50, AML (M2) | C1 (2021.07.26-28) | 4.80×10⁹ | / | 30→43 | SD | |
| | C2 (2021.08.31-02) | 6.27×10⁹ | / | 50 | PD | 5 |
| | C3 (2021.10.23-25) | 4.15×10⁹ | / | UD | NE | |
| | C1 (2021.08.01-03) | 4.40×10⁹ | / | 9.0→20.5 | PD | |
| NO. 012, MALE, 55, AML (M2a) | C2 (2021.09.02-04) | 5.40×10⁹ | combined chemothera py | 67 | PD | 6 |
| | C3 (2021.10.02-04) | 7.02×10⁹ | combined chemothera py | 68 | PD | |
| | C4 (2021.11.10-12) | 6.27×10⁹ | / | UD | NE | |

| | | | | | | |
|---|---|---|---|---|---|---|
| Note: SD, stable disease; PR, partial response; PD, progressive disease; UD, undetected; NE, not evaluable; OS, overall survival (in this report, OS was only calculated up to the data cutoff date 2022.03.01); *, still alive as of the data cutoff date | | | | | | |

### Example 8- IBR733 injection in combination with Venentoclax and Azacitidine for treatment of recurrent or refractory acute myeloid leukemia

In this example, the trial was a single-arm, open-label clinical study investigating the safety, tolerance, and preliminary efficacy of IBR733 injection combined with venentoclax and azacitidine for the treatment of relapsed or refractory acute myeloid leukemia (AML). As of the data cutoff date of April 30, 2022, a total of 3 subjects with relapsed or refractory AML had been enrolled in this study. The ORR after the first treatment cycle reached 100%, with 2 cases of CRi and 1 case of MLFS. The combination treatment and results of efficacy assessment of the subjects in this study are shown in Table 14 below.

**Table 14**

| **Basic information of subjects** | **Administration time** | **Cell count/cycle** | **Blast cells after treatment** (**%)** | **Efficacy assessment** |
|---|---|---|---|---|
| NO. T03001, MALE, 61, REFRACTORY AML (M4) | BASELINE | / | 38.5 | / |
| | C1 (2022.01.14-02.09) | 8×10⁹ | 3.5 | MLFS |
| | C2 (2022.02.22-02.24 ) | 8×10⁹ | 12 | PR |
| | C3 (2022.03.17-03.19) | 8×10⁹ | UD | NE |
| NO. T03002, MALE, 76, REFRACTORY AML (M2) | BASELINE | / | 18.5 | / |
| | C1 (2022.01.12-02.08) | 8×10⁹ | 1 | CRi |
| | C2 (2022.02.22-02.24) | 8×10⁹ | 2 | CRi |
| | C3 (2022.03.17-03.19) | 8×10⁹ | 3 | CRi |
| | C4 (2022.04.29-05.01 ) | 8×10⁹ | The evaluation time had not yet arrived | / |
| NO. T03003, MALE, 61, REFRACTORY AML (M2a) | BASELINE | / | 34.5 | / |
| | C1 (2022.03.31-04.02) | 8×10⁹ | 2.5 | CRi |

| | | | | |
|---|---|---|---|---|
| NOTE: MLFS: Morphological Leukemia-Free State; CRi: Complete Remission with Incomplete Hematological Recovery; UD: Undetected; NE: Not Evaluable. | | | | |

The enrolled patients in both IIT studies were all patients at the extremely advanced stages with recurrent disease that had failed conventional treatments. After one or more cycles of IBR733 injection monotherapy or combination treatment, preliminary clinical efficacy was observed. The existing data on IBR733 injection combined with venentoclax and azacitidine provided a basis for future combination treatment. Previous treatments of multiple relapsed/refractory subjects (NO. 003, 004, and 008 in Example 6, and NO. 001 and 003 in Example 7) showed no benefit from venentoclax combined with azacitidine therapy alone. However, all four relapsed/refractory subjects who received IBR733 injection combined with venentoclax and azacitidine showed good therapeutic benefits (NO. 001 in Example 6 achieved PR, and NO. 001, 002, and 003 in Example 7 achieved MLFS or CRi).

### Example 9 - Evaluation of in vitro killing activities of anti-CD33/CLL1 bispecific antibody-natural killer cell conjugates with different L linkers

An example of the linker conjugated to the antibody moiety in the anti-CD33/CLL1 bispecific antibody-natural killer cell conjugate of the present application is dibenzoazacyclooctyne -glutaryl-amino polyethylene glycol -pentafluorophenyl acetylpiperidinate having the following structure: 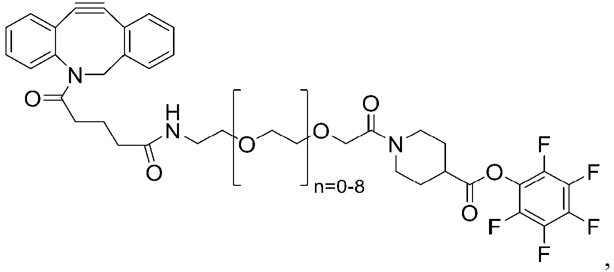 wherein n is an integer of 0-8.

In this example, *in vitro* killing activities of conjugates with three different types of linkers for the antibody moiety: L1 (n=0), L2 (n=3), and L3 (n=1) were tested.

L2 linker is the linker in IBR854 prepared in Example 1 above. Conjugate with L1 linker and conjugated with L3 linker were prepared in a manner similar to that used for preparing IBR733 in Example 1, except that a different L2-2 substrate was used for synthesizing L1 linker and L3 linker. The corresponding substrate was also commercially available.

The assay for the *in vitro* killing activities was similar to that of Example 3. The test cell line was U937. The NK cells (obtained in Step (2) of Example 1) and the UNK cells (obtained in Step (6) of Example 1) were also selected as controls.

As shown by the results in FIG. 22, the conjugates with L1, L2 and L3 linkers exhibited superior cytotoxicity against H292 cells compared with NK and UNK cells, and the conjugate with L2 linker (i.e., IBR733) showed the highest killing activity.

The use of any and all embodiments or exemplary language (e.g., "such as") provided herein is intended only to better illustrate the present application and does not limit the scope of the present application unless otherwise required. The language in the specification should not be construed as indicating that any element not recited in a claim is necessary for the implementation of the inventions of the present application.

All publications and patent applications cited in the present specification are incorporated herein by reference as if each individual publication or patent application were specifically and individually identified by reference. Furthermore, any theory, mechanism, proof, or discovery described herein is intended to further facilitate the understanding of the present application and is not intended to limit the present application in any way to such theory, mechanism, proof, or discovery. Although the present application has been shown and described in detail in the drawings and the foregoing description, the present application should be considered illustrative and not limiting.

## Claims

1. An antibody-natural killer cell (NK cell) conjugate, wherein the antibody is a bispecific antibody comprising a CD33 antigen-binding fragment and a CLL1 antigen-binding fragment, and the bispecific antibody is conjugated to the NK cell via a linker.

2. The antibody-natural killer cell (NK cell) conjugate of claim 1, wherein the CD33 antigen-binding fragment comprises:
HCDR1 as set forth in SEQ ID NO:9 or having at least 80%, 85%, 90%, 95% or 99% identity to the sequence set forth in SEQ ID NO:9,
HCDR2 as set forth in SEQ ID NO: 10 or having at least 80%, 85%, 90%, 95% or 99% identity to the sequence set forth in SEQ ID NO:10,
HCDR3 as set forth in SEQ ID NO:11 or having at least 80%, 85%, 90%, 95% or 99% identity to the sequence set forth in SEQ ID NO:11,
LCDR1 as set forth in SEQ ID NO: 12 or having at least 80%, 85%, 90%, 95% or 99% identity to the sequence set forth in SEQ ID NO:12,
LCDR2 as set forth in SEQ ID NO:13 or having at least 80%, 85%, 90%, 95% or 99% identity to the sequence set forth in SEQ ID NO:13, and
LCDR3 as set forth in SEQ ID NO: 14 or having at least 80%, 85%, 90%, 95% or 99% identity to the sequence set forth in SEQ ID NO:14; and
wherein the amino acid sequences of the HCDRs and the LCDRs are defined according to Kabat.

3. The antibody-natural killer cell (NK cell) conjugate of claim 1 or 2, wherein the amino acid sequence of the heavy chain variable region of the CD33 antigen-binding fragment is set forth in SEQ ID NO:5 or has at least 80%, 85%, 90%, 95% or 99% identity to the sequence set forth in SEQ ID NO:5, and the amino acid sequence of the light chain variable region is set forth in SEQ ID NO:7 or has at least 80%, 85%, 90%, 95% or 99% identity to the sequence set forth in SEQ ID NO:7.

4. The antibody-natural killer cell (NK cell) conjugate of any one of claims 1-3, wherein the CLL1 antigen-binding fragment comprises:
HCDR1 as set forth in SEQ ID NO:15 or having at least 80%, 85%, 90%, 95% or 99% identity to the sequence set forth in SEQ ID NO:15,
HCDR2 as set forth in SEQ ID NO:16 or having at least 80%, 85%, 90%, 95% or 99% identity to the sequence set forth in SEQ ID NO:16,
HCDR3 as set forth in SEQ ID NO: 17 or having at least 80%, 85%, 90%, 95% or 99% identity to the sequence set forth in SEQ ID NO:17,
LCDR1 as set forth in SEQ ID NO:18 or having at least 80%, 85%, 90%, 95% or 99% identity to the sequence set forth in SEQ ID NO:18,
LCDR2 as set forth in SEQ ID NO:19 or having at least 80%, 85%, 90%, 95% or 99% identity to the sequence set forth in SEQ ID NO:19, and
LCDR3 as set forth in SEQ ID NO:20 or having at least 80%, 85%, 90%, 95% or 99% identity to the sequence set forth in SEQ ID NO:20; and
wherein the amino acid sequences of the HCDRs and the LCDRs are defined according to Kabat.

5. The antibody-natural killer cell (NK cell) conjugate of any one of claims 1-4, wherein the amino acid sequence of the heavy chain variable region of the CLL1 antigen-binding fragment is set forth in SEQ ID NO:1 or has at least 80%, 85%, 90%, 95% or 99% identity to the sequence set forth in SEQ ID NO:1, and the amino acid sequence of the light chain variable region is set forth in SEQ ID NO:3 or has at least 80%, 85%, 90%, 95% or 99% identity to the sequence set forth in SEQ ID NO:3.

6. The antibody-natural killer cell (NK cell) conjugate of any one of claims 1-5, wherein the forms of the CD33 antigen-binding fragment and the CLL1 antigen-binding fragment are independently selected from a single chain antibody (scFv) or a Fab fragment.

7. The antibody-natural killer cell (NK cell) conjugate of any one of claims 1-6, wherein the bispecific antibody comprises a CD33 antibody arm comprising the CD33 antigen-binding fragment, and a CLL1 antibody arm comprising the CLL1 antigen-binding fragment, wherein
the CD33 antibody arm comprises a heavy chain variable region set forth in SEQ ID NO:5, a heavy chain constant region set forth in SEQ ID NO:6, a light chain variable region set forth in SEQ ID NO:7, and a light chain constant region set forth in SEQ ID NO:8; and/or
the CLL1 antibody arm comprises a heavy chain variable region set forth in SEQ ID NO: 1, a heavy chain constant region set forth in SEQ ID NO:2, a light chain variable region set forth in SEQ ID NO:3, and a light chain constant region set forth in SEQ ID NO:4.

8. The antibody-natural killer cell (NK cell) conjugate of any one of claims 1-7, wherein:
the bispecific antibody is an intact antibody; and/or
the bispecific antibody is a humanized antibody or a fully human antibody; and/or
the bispecific antibody is a monoclonal antibody; and/or
the bispecific antibody is of IgG1, IgG2 or IgG4 isotype; and/or
the bispecific antibody comprises a light chain constant region of κ subtype.

9. The antibody-natural killer cell (NK cell) conjugate of any one of claims 1-8, wherein the NK cell is CD16⁺ and/or NKG2D⁺, preferably CD16⁺NKG2D⁺.

10. The antibody-natural killer cell (NK cell) conjugate of any one of claims 1-9, wherein the conjugate comprises at least 90% of CD16⁺NKG2D⁺NK cells, preferably, meanwhile the conjugate comprises at least 95% of CD56⁺NK cells.

11. The antibody-natural killer cell (NK cell) conjugate of any one of claims 1-10, wherein
the NK cell is obtained from *in vitro* culture and expansion of NK cells derived from peripheral blood mononuclear cells (PBMCs); or
the NK cell is obtained from *in vitro* culture and expansion of NK cells derived from umbilical cord blood; or
the NK cell is obtained from *in vitro* induction, culture and expansion of inducible pluripotent stem cells (iPSCs) or mesenchymal stem cells (ESCs).

12. The antibody-natural killer cell (NK cell) conjugate of any one of claims 1-11, wherein the bispecific antibody is conjugated to the NK cell via a click chemical reaction of linkers.

13. The antibody-natural killer cell (NK cell) conjugate of claim 12, wherein the bispecific antibody is conjugated to the NK cell via a first linker conjugated to the bispecific antibody and a second linker conjugated to the NK cell, with the first linker and the second linker conjugated to each other to form the antibody-NK cell conjugate.

14. The antibody-natural killer cell (NK cell) conjugate of claim 13, wherein the first linker is an active ester capable of forming a conjugation to a lysine residue of the antibody by a reaction converting an ester bond into an amide bond, e.g., the active ester is a pentafluorophenyl ester, such as pentafluorophenyl piperidinate.

15. The antibody-natural killer cell (NK cell) conjugate of claim 14, wherein the first linker further comprises a carbon-carbon triple bond structure capable of undergoing a cyclization reaction with an azide group to form a five-membered ring of triazole, e.g., the carbon-carbon triple bond structure is an octyne group.

16. The antibody-natural killer cell (NK cell) conjugate of claim 15, wherein the first linker is a dibenzoazacyclooctyne-glutaryl-amino polyethylene glycol-pentafluorophenyl acetylpiperidinate having the following structure: 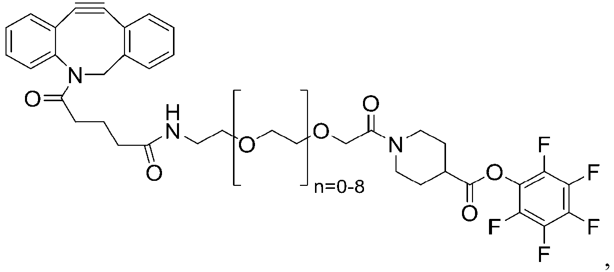 wherein n is an integer of 0-8.

17. The antibody-natural killer cell (NK cell) conjugate of claim 16, wherein the first linker is dibenzoazacyclooctyne-glutaryl-amino tetraethylene glycol-pentafluorophenyl acetylpiperidinate having the following structure:

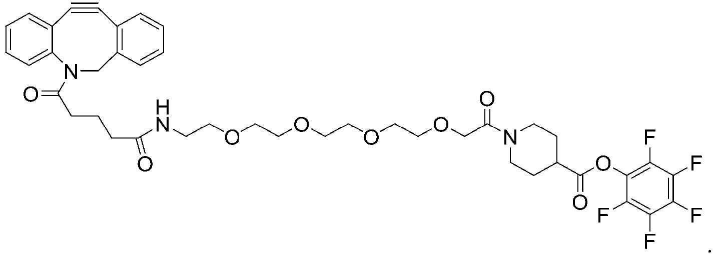

18. The antibody-natural killer cell (NK cell) conjugate of claim 13, wherein the second linker is an azidoacetylcyclohexosamine, e.g., an azidoacetylcyclogalactosamine or an azidoacetylglucosamine.

19. The antibody-natural killer cell (NK cell) conjugate of claim 18, wherein the second linker is 1,3,4,6-oxo-tetraacetyl-2-azidoacetamide-2-deoxy-a,b-D-galactose having the following structure:

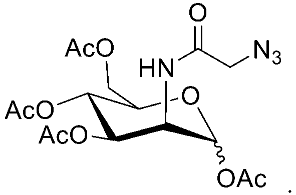

20. The antibody-natural killer cell (NK cell) conjugate of claim 19, wherein the second linker comprises at least 90% of the structure having a single α or β configuration.

21. A cell population comprising the antibody-natural killer cell (NK cell) conjugate of any one of claims 1-20.

22. The cell population of claim 21, wherein the count of CD3⁻CD56⁺CD16⁺ cells is at least 95%, preferably at least 98% with respect to the total cell count in the cell population, and/or the count of CD3⁻CD56⁺NKG2D⁺ cells is at least 95%, preferably at least 98% with respect to the total cell count in the cell population.

23. The cell population of claim 21, wherein:
the count of CD3⁺CD56⁺ cells is no more than 5% with respect to the total cell count in the cell population; and/or
the count of CD3⁻CD19⁺ cells is no more than 2% with respect to the total cell count in the cell population; and/or
the counts of CD3⁺CD4⁺ and CD3⁺CD8⁺ cells are no more than 2% with respect to the total cell count in the cell population.

24. The cell population of any one of claims 21-23, wherein, by cell count, the antibody-natural killer cell (NK cell) conjugate is at least 90%, preferably at least 95%, more preferably at least 98%, most preferably at least 99% with respect to the total cell count in the cell population.

25. A pharmaceutical composition comprising the antibody-natural killer cell (NK cell) conjugate of any one of claims 1-20 or the cell population of any one of claims 21-24, and a pharmaceutically acceptable carrier.

26. The pharmaceutical composition of claim 25, comprising sodium chloride and/or human serum albumin.

27. The pharmaceutical composition of claim 25 or 26, comprising trehalose, sucrose, dextran, DMSO, or any combination thereof.

28. The pharmaceutical composition of any one of claims 25-27, for use in the treatment of acute myeloid leukemia (AML) in an individual.

29. The pharmaceutical composition of claim 28, for use in the treatment of an initially diagnosed acute myeloid leukemia.

30. The pharmaceutical composition of claim 28, for use in the treatment of relapsed or refractory acute myeloid leukemia.

31. The pharmaceutical composition of claim 30, wherein the individual has received a chemotherapy and/or a targeted drug therapy.

32. The pharmaceutical composition of claim 31, wherein the targeted drug is gilteritinib and/or venentoclax.

33. The pharmaceutical composition of any one of claims 25-32, for use in combination with venentoclax and/or azacitidine for the treatment of relapsed or refractory acute myeloid leukemia.

34. Use of the antibody-natural killer cell (NK cell) conjugate of any one of claims 1-20 or the cell population of any one of claims 21-24 in the manufacture of a medicament for the treatment of acute myeloid leukemia (AML).

35. The use of claim 34, wherein the medicament is for the treatment of an initially diagnosed acute myeloid leukemia.

36. The use of claim 34, wherein the medicament is for the treatment of relapsed or refractory acute myeloid leukemia.

37. The use of claim 36, wherein the individual has received a chemotherapy and/or a targeted drug therapy.

38. The use of claim 37, wherein the targeted drug is gilteritinib and/or venentoclax.

39. The use of any one of claims 34-38, wherein the medicament is for use in combination with venentoclax and/or azacitidine for the treatment of relapsed or refractory acute myeloid leukemia.

40. A method of treating acute myeloid leukemia (AML) in an individual, the method comprising administering to the individual an effective amount of the antibody-natural killer cell (NK cell) conjugate of any one of claims 1-20 or the cell population of any one of claims 21-24 or the pharmaceutical composition of any one of claims 25-33.

41. The method of claim 40, wherein the individual suffers from an initially diagnosed acute myeloid leukemia.

42. The method of claim 40, wherein the individual suffers from relapsed or refractory acute myeloid leukemia.

43. The method of claim 42, wherein the individual has received a chemotherapy and/or a targeted drug therapy.

44. The method of claim 43, wherein the targeted drug is gilteritinib and/or venentoclax.

45. The method of any one of claims 40-44, further comprising administering to the individual venentoclax and/or azacitidine for combination treatment of relapsed or refractory acute myeloid leukemia.
